# EUROPEAN PATENT SPECIFICATION

(11) **EP 2 874 643 B1**
(45) Date of publication and mention of the grant of the patent: **26.06.2019**
(21) Application number: 12873353.2
(22) Date of filing: 31.03.2012
(51) Int. Cl.: A61K 38/17, A61K 39/395, A61K 31/7088, C07K 16/46, C12N 15/85, A61P 19/00, C07K 14/705

(54) **OSTEOPROTEGERIN DERIVED COMPOSITION AND USE THEREOF**
AUS OSTEOPROTEGERIN GEWONNENE ZUSAMMENSETZUNG UND VERWENDUNG DAVON
COMPOSITION DÉRIVÉE DE L'OSTÉOPROTÉGÉRINE ET SON UTILISATION

(43) Date of publication of application: 27.05.2015
(73) Proprietor: R-Pharm International, LLC, 123154 Moscow (RU)
(72) Inventor: LAVROVSKY, Yan, San Diego, CA 92121 (US); XU, Ting, Needham, MA 02492 (US); REPIK, Alexey, Moscow 121609 (RU); GUO, Kangping, Jiangsu (CN); SAMSONOV, Mikhail, Moscow 125313 (RU); IGNATIEV, Vasily, Moscow 121353 (RU)
(74) Representative: HGF Limited
(86) International application number: PCT/US2012/031737
(87) International publication number: WO 2013/147899

(56) References cited:
- WO-A1-01/18203
- WO-A1-97/23614
- WO-A2-01/17543
- WO-A2-02/064782
- SIMONET W S ET AL: "Osteoprotegerin: A novel secreted protein involved in the regulation of bone density", CELL, vol. 89, no. 2, 18 April 1997 (1997-04-18) , pages 309-319, XP002077048, ISSN: 0092-8674, DOI: 10.1016/S0092-8674(00)80209-3
- ZAULI G ET AL: "Role of full-length osteoprotegerin in tumor cell biology", CELLULAR AND MOLECULAR LIFE SCIENCES, vol. 66, no. 5, March 2009 (2009-03), pages 841-851, XP002754221, ISSN: 1420-682X, DOI: 10.1007/s00018-008-8536-x
- MORONY S ET AL: "A CHIMERIC FORM OF OSTEOPROTEGERIN INHIBITS HYPERCALCEMIA AND BONE RESORPTION INDUCED BY IL-1BETA, TNF-ALPHA, PTH, PTHRP, AND 1,25(OH)2D3", JOURNAL OF BONE AND MINERAL RESEARCH, BLACKWELL SCIENCE, INC, US, vol. 14, no. 9, 1 September 1999 (1999-09-01), pages 1478-1485, XP000971493, ISSN: 0884-0431, DOI: 10.1359/JBMR.1999.14.9.1478
- DATABASE Genebank [Online] 2 December 1998 (1998-12-02), Takahasi n et al: "Immunoglobulin gamma-1 heavy chain constant region", XP002754224, retrieved from NCBI Database accession no. AAC82527
- DATABASE Genebank [Online] 2 December 1998 (1998-12-02), Takahasi net al: "Homo sapiens immunoglobulin gamma-1 heavy chain constant region (IGHG1) gene, partial cds", XP002754225, retrieved from NCBI Database accession no. J00228
- DATABASE Genebank [Online] 27 April 1993 (1993-04-27), Lewis A.P. et al: "IgG homo sapiens", XP002754226, retrieved from NCBI Database accession no. AAA02914
- DATABASE GENBANK [Online] 11 December 2011 XP003032697 Database accession no. AEV43323.1
- SORDILLO, E.M. ET AL.: 'RANK-Fc: A Therapeutic Antagonist for RANK-L in Myeloma.' CANCER SUPPLEMENT vol. 97, no. 3, 2003, pages 802 - 812, XP002967812
- BEKKER PIROW J ET AL: "The effect of a single dose of osteoprotegerin in postmenopausal women", JOURNAL OF BONE AND MINERAL RESEARCH, vol. 16, no. 2, February 2001 (2001-02), pages 348-360, ISSN: 0884-0431
- BODY JEAN-JACQUES ET AL: "A phase I study of AMGN-0007, a recombinant osteoprotegerin construct, in patients with multiple myeloma or breast carcinoma related bone metastases.", CANCER, vol. 97, no. 3 Supplement, 1 February 2003 (2003-02-01), pages 887-892, ISSN: 0008-543X

## Description

### FIELD OF THE INVENTION

Generally, the invention relates to the field of biological pharmaceuticals as well as their use in conditions associated with bone resorption, for example in oncology. More specifically, the invention relates to an osteoprotegerin-derived composition that binds to receptor activator of NF-kappaB ligand (RANKL).

### BACKGROUND

The approaches described in this section could be pursued, and are not necessarily approaches that have previously been conceived or pursued. Therefore, unless otherwise indicated, it should not be assumed that any of the approaches described in this section qualify as prior art, merely by virtue of their inclusion into this section.

Human osteoprotegerin (OPG; GenBank: U94332.1) is a 401 amino acid protein which contains a signal peptide of 21 amino acids, that is cleaved before glutamic acid 22 giving rise to a mature soluble protein of 380 amino acid. OPG is a member of the tumor necrosis factor receptor (TNFR) family, comprising four cysteine-rich TNFR like domains in its N-terminal portion. OPG has been shown to have a role in the development of bone, and mice lacking the OPG gene had an osteoporotic phenotype and gross skeletal abnormalities.

OPG, which is produced by osteoblasts and bone marrow stromal cells, acts as a secreted decoy receptor with no apparent direct signaling function. OPG acts by binding to its natural ligand - osteoprotegerin ligand (OPGL), which is also known as RANKL (receptor activator of NF-kappaB ligand). The binding between OPG and RANKL prevents RANKL from activating its cognate receptor RANK, which is an osteoclast receptor vital for osteoclast differentiation, activation and survival.

Recombinant OPG exists in monomeric and dimeric forms of apparent molecular weights of about 55 kDa and about 110 kDa, respectively. Truncation of the N-terminal domain to residue cysteine 185 results in OPG inactivation, presumably by disrupting a disulfide bond of the TNFR-like domain, whereas truncation of the C-terminal portion of the protein to residue 194 does not alter biological activity.

Overexpression of OPG in transgenic mice leads to profound osteopetrosis characterized by a near complete lack of osteoclasts in the mice. Conversely, ablation of the OPG gene causes severe osteoporosis in mice, indicating an important physiological role of OPG in regulating bone resorption. The secretion of OPG and RANKL from osteoblasts and stromal cells is regulated by numerous hormones and cytokines. The relative levels of OPG and RANKL production are thought to control the extent of bone resorption: expression of RANKL increases bone resorption, whereas excess OPG has the opposite effect. Recombinant OPG blocks the effects of the vast majority of the factors which stimulate osteoclasts, in vitro and in vivo. OPG also inhibits bone resorption in a variety of animal disease models, including ovariectomy, induced osteoporosis, humoral hypercalcemia of malignancy, and experimental bone metastasis. Therefore, OPG might represent an effective therapeutic option for diseases associated with excessive osteoclast activity (Kostenuik PJ, Shalhoub V., Curr Pharm Des. 2001 May;7(8):613-35).

Denosumab is a high affinity fully human monoclonal antibody that binds to human RANKL and inhibits its interactions with RANK, thus having a similar to OPG mode of action. Denosumab under the trade name Prolia was approved by U.S. Food and Drug Administration (FDA) for prevention and treatment of osteoporosis in postmenopausal women. Denosumab under the trade name Xgeva was approved by U.S. Food and Drug Administration (FDA) for the prevention of skeletal-related events in patients with bone metastases from solid tumors. Further clinical trials of denosumab for other bone remodeling related conditions are currently under way, i.e. for bone metastases from other forms of cancer.

It would therefore be desirable to have a therapeutic composition that is capable of binding to RANKL and is based on the naturally occurring OPG molecule, which, while having an acceptable pharmacological profile, has a broader therapeutic potential.

### SUMMARY OF THE INVENTION

The invention is defined by the appended claims.

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

In certain aspects, the present invention provides for a polypeptide that binds human RANKL, the polypeptide comprising a first amino acid sequence comprising amino acids 1 through 215 of human osteoprotegerin (GenBank: U94332.1) and a second amino acid sequence comprising amino acids 103 through 329 of human immunoglobulin gamma-1 Fc (GenBank: J00228.1). The polypeptide is capable of inhibiting RANKL-induced osteoclast differentiation in an in vitro cell-based assay with an EC50 of about 50 ng/ml or to no more than 10% at a concentration of 100 ng/ml. The polypeptide may comprise amino acid sequence of SEQ ID NO. 1.

The closest prior art to the molecule of the invention, for which clinical results are available, is a fusion molecule containing OPG[22-194] fragment linked at at the C-terminus to human IgG1-Fc portion via a flexible linker known as AMGN-0007. The results of a clinical phase I study for the molecule are presented in J.-J. Body et al "CANCER supplement, Vol. 97, no.3, pp.887-892 (2003)".

In certain aspects of the disclosure a therapeutic composition comprising a polypeptide that binds to human RANKL is provided, the polypeptide comprising a biologically active portion of human osteoprotegerin and a Fc portion of human immunoglobulin gamma-1. The polypeptide is substantially purified to a homo-dimeric form in solution. The polypeptide may exhibit a half-life in systemic circulation in mice of at least 80 hours after a subcutaneous administration at a dose of 5 mg/kg. The polypeptide may be capable of substantially inhibiting bone lysis induced by human breast cancer in a murine bone lytic model for at least 6 weeks in a course of subcutaneous administration of said composition at a dose of no more than 1 mg/kg three times weekly.

In certain aspects, the present invention provides for a therapeutic composition comprising a first amino acid sequence comprising amino acids 1 through 215 of human osteoprotegerin and a second amino acid sequence comprising amino acids 103 through 329 of human immunoglobulin gamm-1 Fc, wherein the polypeptide is purified to a homo-dimeric form in solution to no less than 99%. The polypeptide may exhibit a half-life in
systemic circulation in mice of at least 80 hours after a subcutaneous administration at a dose of 5 mg/kg. The polypeptide may be capable of substantially inhibiting bone lysis induced by human breast cancer in a murine bone lytic model for at least 6 weeks in a course of subcutaneous administration of said composition at a dose of no more than 1 mg/kg three times weekly. The polypeptide may comprise amino acid sequence of SEQ ID NO. 1.

In certain aspects, the present invention provides for an isolated nucleic acid encoding a polypeptide that binds to human RANKL as provided by the claims. The polypeptide comprises a first amino acid sequence comprising amino acids 1 through 215 of human osteoprotegerin. The polypeptide further comprises a second amin acid sequence comprising amino acids 103 through 329 of human immunoglobulin
gamma-1 Fc. The first amino acid sequence in the polypeptide may precede the second amino acid sequence. The polypeptide may comprise amino acid sequence of SEQ ID NO. 1. The nucleic acid may have its codon usage optimized for high expression of the polypeptide in a mammalian cell. The nucleic acid may comprise the sequence of SEQ ID NO. 2. The nucleic acid may comprise an expression vector.

In certain aspects, the present invention provides for a heterologous expression system. The expression system harbors an expression vector comprising a nucleic acid sequence encoding a polypeptide that binds to human RANKL as provided by the claims. The polypeptide comprises a first amino acid sequence comprising amino acids 1 through 215 of human osteoprotegerin. The polypeptide further comprises a second amino acid sequence comprising amino acids 103 through 329 of human immunoglobulin gamma-1 Fc. The first amino acid sequence in the polypeptide may precede the second amino acid sequence. The polypeptide may comprise amino acid sequence of SEQ ID NO. 1. The expression vector of the expression system may be harbored in a mammalian cell. The mammalian cell may be a Chinese hamster ovaries (CHO) cell. The expression level of the polypeptide in the expression system may be at least 125 mg per liter of cell culture.

In certain aspects, the present disclosure provides for a use of a substance for manufacture of a medicament for the treatment or prevention of a disease associated with bone resorption or remodeling. The substance comprises a polypeptide that binds to human RANKL. The polypeptide comprises a first amino acid sequence comprising amino acids 1 through 215 of human osteoprotegerin. The polypeptide further comprises a second amino acid sequence comprising amino acids 103 through 329 of human immunoglobulin gamma-1 Fc. The first amino acid sequence in the polypeptide may precede the second amino acid sequence. The polypeptide may comprise amino acid sequence of SEQ ID NO. 1. The disease associated with bone resorption or remodeling may be a carcinoma, a breast cancer, a prostate cancer, multiple myeloma, a bone sarcoma, bone metastases due to solid tumors, osteoporosis, rheumatoid arthritis, or psoriatic arthritis.

In certain aspects, the present disclosure provides for a method of treating or preventing a disease associated with bone resorption or remodeling. The method comprises administering to a patient in need for treating or preventing a disease associated with bone resorption or remodeling a therapeutically effective amount of a pharmaceutical composition comprising a polypeptide that binds to human RANKL. The polypeptide comprises a first amino acid sequence comprising amino acids 1 through 215 of human osteoprotegerin. The polypeptide further comprises a second amino acid sequence comprising amino acids 103 through 329 of human immunoglobulin gamma-1 Fc. The first amino acid sequence in the polypeptide may precede the second amino acid sequence. The polypeptide may comprise amino acid sequence of SEQ ID NO. 1. The disease associated with bone resorption or remodeling may be a carcinoma, a breast cancer, a prostate cancer, multiple myeloma, a bone sarcoma, bone metastases due to solid tumors, osteoporosis, rheumatoid arthritis, or psoriatic arthritis.

These and other aspects and advantages of the invention described herein will become apparent upon consideration of the Figures and detailed description below.

### BRIEF DESCRIPTION OF THE DRAWINGS

The following drawings and descriptions are provided to aid in the understanding of the invention:
**Figure 1** schematically shows the map of pCDNA4-Fc plasmid and annotated sequence used in the cloning of the polypeptides of the present invention;
**Figure 2** schematically shows the map of pKN002 plasmid and annotated sequence used in the cloning of the polypeptides of the present invention;
**Figure 3** shows representative transfection growth curves obtained in the process of generating stable cell lined for expressing the polypeptides of the present invention;
**Figure 4** shown a size-exclusion HPLC analytical chromatogram of the sample containing polypeptide of SEQ ID NO. 1 after the anion exchange chromatography purification step;
**Figure 5** shown a SDS-PAGE analysis (under reducing conditions) of the sample containing polypeptide of SEQ ID NO. 1 after the anion exchange chromatography purification step;
**Figure 6** shown a hydroxylapatite chromatography elution profiled showing superior resolution of the sample containing polypeptide of SEQ ID NO. 1;
**Figure 7** shown a size-exclusion HPLC analytical chromatogram of the sample of the main peak of the eluate containing polypeptide of SEQ ID NO. 1 from the hydroxylapatite chromatography;
**Figure 8** shows X-ray images of tibiae of representative mice from group 1 (s.c. administration of formulation buffer) at eight weeks post-inoculation used for assessment of bone lysis (assigned scores are indicated below each image);
**Figure 9** shows X-ray images of tibiae of representative mice from group 2 (s.c. administration of polypeptide of SEQ ID NO.1 at 1 mg/kg) at eight weeks post-inoculation used for assessment of bone lysis (assigned scores are indicated below each image);
**Figure 10** shows two-dimensional microCT scan images for one representative mouse in each of groups 1, 2 and 5; and
**Figure 11** shows three-Dimensional microCT scan images for one representative mouse in each of groups 1, 2 and 5.

### DETAILED DESCRIPTION OF THE INVENTION

The teachings disclosed herein are based, in part, upon engineering of a protein molecule comprising a biologically active N-terminal portion of OPG which is fused to the Fc portion of a human IgG. To enable recombinant production of such OPG-derived protein molecule, a DNA expression vector has been constructed for overproducing the protein molecule in a heterologous protein expression system, and mammalian cells have been prepared stably expressing the protein molecule to a high expression level. Unexpectedly, the protein molecule from the recombinant source formed homo-dimmers and homo-tetramers in solution. A protein purification procedure has been devised allowing obtaining a physiologically relevant substantially pure homo-dimeric preparation of the protein molecule. Thus purified protein molecule demonstrates a high degree of inhibition of RANKL-induced osteoclast differentiation in a relevant *in vitro* cell-based assay. Unexpectedly, the protein molecule exhibits an acceptable pharmacokinetics profile upon subcutaneous animal administration, while not showing any adverse toxicity or immunogenicity. The protein molecule also demonstrates a high degree of efficacy in a predictive animal model of bone metastasis induced by human breast cancer.

The terms used in this specification generally have their ordinary meanings in the art, within the context of this invention and in the specific context where each term is used. Certain terms are discussed below or elsewhere in the specification, to provide additional guidance to the practitioner in describing the compositions and methods of the invention and how to make and use them. The scope or meaning of any use of a term will be apparent from the specific context in which the term is used. "About" and "approximately" shall generally mean an acceptable degree of error for the quantity measured given the nature or precision of the measurements. Typically, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 5- fold and more preferably within 2-fold of a given value. Numerical quantities given herein are approximate unless stated otherwise, meaning that the term "about" or "approximately" can be inferred when not expressly stated.

The methods of the invention may include steps of comparing sequences to each other, including wild-type sequence to one or more mutants (sequence variants). Such comparisons typically comprise alignments of polymer sequences, e.g., using sequence alignment programs and/or algorithms that are well known in the art (for example, BLAST, FASTA and MEGALIGN, to name a few). The skilled artisan can readily appreciate that, in such alignments, where a mutation contains a residue insertion or deletion, the sequence alignment will introduce a "gap" (typically represented by a dash, or "A") in the polymer sequence not containing the inserted or deleted residue.

The methods of the invention may include statistical calculations, e.g. determination of IC50 or EC50 values, etc.. The skilled artisan can readily appreciate that such can be performed using a variety of commercially available software, e.g. PRISM (GraphPad Software Inc, La Jolla, CA, USA) or similar.

"Homologous," in all its grammatical forms and spelling variations, refers to the relationship between two proteins that possess a "common evolutionary origin," including proteins from superfamilies in the same species of organism, as well as homologous proteins from different species of organism. Such proteins (and their encoding nucleic acids) have sequence homology, as reflected by their sequence similarity, whether in terms of percent identity or by the presence of specific residues or motifs and conserved positions. However, in common usage and in the instant application, the term "homologous," when modified with an adverb such as "highly," may refer to sequence similarity and may or may not relate to a common evolutionary origin.

The term "sequence similarity," in all its grammatical forms, refers to the degree of identity or correspondence between nucleic acid or amino acid sequences that may or may not share a common evolutionary origin.

The terms "protein" and "polypeptide" are used interchangeably. In general, OPG-derived proteins of the present teachings for use in mammals are expressed in mammalian cells that allow for proper post-translational modifications, such as CHO or HEK293 cell lines, although other mammalian expression cell lines are expected to be useful as well. It is therefore anticipated that the OPG-derived proteins may be post-translationally modified without substantially effecting their biological function.

In certain aspects, functional variants of OPG-derived protein molecules of the present teachings include fusion proteins having at least a biologically active portion of the human OPG and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (e.g., an Fc), maltose binding protein (MBP), or human serum albumin. A fusion domain may be selected so as to confer a desired property. For example, the OPG polypeptide portion may be fused with a domain that stabilizes the OPG polypeptide *in vivo* (a "stabilizer" domain), optionally via a suitable peptide linker. The term "stabilizing" means anything that increases the half life of a polypeptide in systemic circulation, regardless of whether this is because of decreased destruction, decreased clearance, or other pharmacokinetic effect. Fusions with the Fc portion of an immunoglobulin are known to confer desirable pharmacokinetic properties on certain proteins. Likewise, fusions to human serum albumin can confer desirable properties. Other types of fusion domains that may be selected include multimerizing (e.g., dimerizing, tetramerizing) domains and functional domains that confer an additional biological function, e.g. promoting accumulation at the targeted site of action *in vivo.*

In certain aspects, the present invention provides for a polypeptide comprising the leading 215 amino acids of the human OPG (GenBank: U94332.1), followed by 227 amino acids of the Fc portion of the human Ig Gamma-1 (GenBank: J00228.1). In an example embodiment, the protein molecule of the present invention comprises amino acid sequence of SEQ ID NO. 1.
hOPG-hIgG1-Fc polypeptide (SEQ ID NO.1)

MNKLLCCALV FLDISIKWTT QETFPPKYLH YDEETSHQLL CDKCPPGTYL KQHCTAKWKT 60
VCAPCPDHYY TDSWHTSDEC LYCSPVCKEL QYVKQECNRT HNRVCECKEG RYLEIEFCLK 120
HRSCPPGFGV VQAGTPERNT VCKRCPDGFF SNETSSKAPC RKHTNCSVFG LLLTQKGNAT 180
HDNICSGNSE STQKCGIDVT LCEEAFFRFA VPTKFDKTHT CPPCPAPELL GGPSVFLFPP 240
KPKDTLMISR TPEVTCVVVD VSHEDPEVKF NWYVDGVEVH NAKTKPREEQ YNSTYRVVSV 300
LTVLHQDWLN GKEYKCKVSN KALPAPIEKT ISKAKGQPRE PQVYTLPPSR DELTKNQVSL 360
TCLVKGFYPS DIAVEWESNG QPENNYKTTP PVLDSDGSFF LYSKLTVDKS RWQQGNVFSC 420
SVMHEALHNH YTQKSLSLSP GK 442

In certain aspects, the present invention provides for a recombinant DNA molecule having an open reading frame coding for a polypeptide comprising the leading 215 amino acids of the human OPG followed by 227 amino acids of the Fc portion of the human Ig Gamma-1, optionally connected via a flexible linker. In an example embodiment, the recombinant DNA molecule of the present invention comprises nucleotide sequence of SEQ ID NO. 2.
hOPG-hIgG1-Fc DNA (SEQ ID NO. 2)

ATGAATAAGC TGCTGTGCTG TGCCCTCGTG TTTCTCGATA TAAGCATTAA GTGGACTACC 60
CAGGAGACAT TCCCTCCTAA GTATCTGCAC TATGACGAGG AGACAAGCCA TCAGCTGCTG 120
TGCGATAAGT GTCCTCCTGG GACCTATCTC AAACAACATT GTACAGCCAA ATGGAAGACA 180
GTCTGCGCTC CATGTCCTGA CCACTACTAC ACCGACTCTT GGCATACTAG CGACGAATGT 240
CTGTATTGTT CACCCGTGTG CAAGGAGCTG CAATACGTGA AACAGGAATG CAATAGGACA 300
CATAACCGCG TGTGTGAATG CAAAGAGGGC AGGTATCTGG AGATCGAATT TTGTCTGAAG 360
CACCGGAGCT GCCCACCCGG CTTTGGAGTG GTCCAGGCCG GGACTCCCGA GAGAAACACT 420
GTGTGCAAAA GATGCCCAGA CGGATTCTTT TCAAACGAGA CATCTTCTAA GGCACCATGT 480
CGGAAGCACA CTAACTGTTC CGTCTTTGGG CTGCTGCTCA CCCAGAAGGG CAATGCCACC 540
CACGATAATA TTTGCTCCGG AAACTCCGAA TCCACCCAAA AGTGCGGGAT AGATGTTACC 600
CTCTGCGAAG AGGCATTCTT CCGCTTCGCT GTTCCTACCA AGTTCGACAA AACTCACACA 660
TGCCCACCGT GCCCAGCTCC GGAACTCCTG GGCGGACCGT CAGTCTTCCT CTTCCCCCCA 720
AAACCCAAGG ACACCCTCAT GATCTCCCGG ACCCCTGAGG TCACATGCGT GGTGGTGGAC 780
GTGAGCCACG AAGACCCTGA GGTCAAGTTC AACTGGTACG TGGACGGCGT GGAGGTGCAT 840
AATGCCAAGA CAAAGCCGCG GGAGGAGCAG TACAACAGCA CGTACCGTGT GGTCAGCGTC 900
CTCACCGTCC TGCACCAGGA CTGGCTGAAT GGCAAGGAGT ACAAGTGCAA GGTCTCCAAC 960
AAAGCCCTCC CAGCCCCCAT CGAGAAAACC ATCTCCAAAG CCAAAGGGCA GCCCCGAGAA 1020
CCACAGGTGT ACACCCTGCC CCCATCCCGG GATGAGCTGA CCAAGAACCA GGTCAGCCTG 1080
ACCTGCCTGG TCAAAGGCTT CTATCCCAGC GACATCGCCG TGGAGTGGGA GAGCAATGGG 1140
CAGCCGGAGA ACAACTACAA GACCACGCCT CCCGTGTTGG ACTCCGACGG CTCCTTCTTC 1200
CTCTACAGCA AGCTCACCGT GGACAAGAGC AGGTGGCAGC AGGGGAACGT CTTCTCATGC 1260
TCCGTGATGC ATGAGGCTCT GCACAACCAC TACACGCAGA AGAGCCTCTC CCTGTCTCCG 1320
GGTAAA 1326

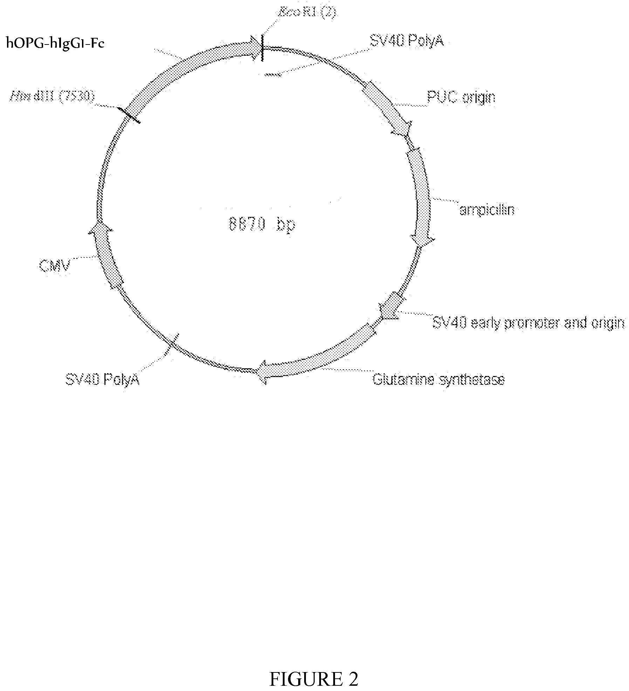

In certain aspects, the present invention provides for a recombinant mamalian expression plasmid for high expression of a polypeptide comprising the leading 215 amino acids of the human OPG followed by 227 amino acids of the Fc portion of the human Ig Gamma-1, optionally connected via a flexible linker. This plasmid comprises the cytomegalovirus (CMV) promoter to drive transcription of the gene coding for said polypeptide, followed by the bGH polyadenylation and transcription termination sequence. The plasmid also contains a pUC origin of replication and β-lactamase gene, which confers ampicillin resistance, for supporting plasmid propagation and selection in bacteria. The plasmid further contains a gene for Glutamine synthetase, a selectable marker widely used for establishing stable CHOK1 and NSO cell lines. The plasmid of the present invention is illustratively shown in **Figure 2****.**

In an example embodiment, the mammalian expression plasmid of the present invention comprises nucleotide sequence of SEQ ID NO. 3.
hOPG-hIgG1-Fc expression plasmid (SEQ ID NO.3)

GAATTCATTG ATCATAATCA GCCATACCAC ATTTGTAGAG GTTTTACTTG CTTTAAAAAA 60
CCTCCCACAC CTCCCCCTGA ACCTGAAACA TAAAATGAAT GCAATTGTTG TTGTTAACTT 120
GTTTATTGCA GCTTATAATG GTTACAAATA AAGCAATAGC ATCACAAATT TCACAAATAA 180
AGCATTTTTT TCACTGCATT CTAGTTGTGG TTTGTCCAAA CTCATCAATG TATCTTATCA 240
TGTCTGGCGG CCGCGAGACG CCATCCACGC TGTTTTGACC TCCATAGAAG ACACCGGGAC 300
CGATCCAGCC TCCGCGGCCG GGAACGGTGC ATTGGAACGC GGATTCCCCG TGCCAAGAGT 360
GACGTAAGTA CCGCCTATAG AGTCTATAGG CCCACCCCCT TGGCTTCTTA TGCATGCTAT 420
ACTGTTTTTG GCTTGGGGTC TATACACCCC CGCTTCCTCA TGTTATAGGT GATGGTATAG 480
CTTAGCCTAT AGGTGTGGGT TATTGACCAT TATTGACCAC TCCCCTATTG GTGACGATAC 540
TTTCCATTAC TAATCCATAA CATGGCTCTT TGCCACAACT CTCTTTATTG GCTATATGCC 600
AATACACTGT CCTTCAGAGA CTGACACGGA CTCTGTATTT TTACAGGATG GGGTCTCATT 660
TATTATTTAC AAATTCACAT ATACAACACC ACCGTCCCCA GTGCCCGCAG TTTTTATTAA 720
ACATAACGTG CTCCACGCGA ATCTCGGGTA CGTGTTCCGG ACATGGGCTC TTCTCCGGTA 780
GCGGCGGAGC TTCTACATCC GAGCCCTGCT CCCATGCCTC CAGCGACTCA TGGTCGCTCG 840
GCAGCTAGTG GAGGCCAGAC TTAGGCACAG CACGATGCCC ACCACCACCA GTGTGCCGCA 900
CAAGGCCGTG GCGGTAGGGT ATGTGTCTGA AAATGAGCTC GGGGAGCGGG CTTGCACCAA 960
AAATTTTCGC GTCGACTATA CCGTCCACAT GTGAGCAAAA GGCCAGCAAA AGGCCAGGAA 1020
CCGTAAAAAG GCCGCGTTGC TGGCGTTTTT CCATAGGCTC CGCCCCCCTG ACGAGCATCA 1080
CAAAAATCGA CGCTCAAGTC AGAGGTGGCG AAACCCGACA GGACTATAAA GATACCAGGC 1140
GTTTCCCCCT GGAAGCTCCC TCGTGCGCTC TCCTGTTCCG ACCCTGCCGC TTACCGGATA 1200

CCTGTCCGCC TTTCTCCCTT CGGGAAGCGT GGCGCTTTCT CATAGCTCAC GCTGTAGGTA 1260
TCTCAGTTCG GTGTAGGTCG TTCGCTCCAA GCTGGGCTGT GTGCACGAAC CCCCCGTTCA 1320
GCCCGACCGC TGCGCCTTAT CCGGTAACTA TCGTCTTGAG TCCAACCCGG TAAGACACGA 1380
CTTATCGCCA CTGGCAGCAG CCACTGGTAA CAGGATTAGC AGAGCGAGGT ATGTAGGCGG 1440
TGCTACAGAG TTCTTGAAGT GGTGGCCTAA CTACGGCTAC ACTAGAAGAA CAGTATTTGG 1500
TATCTGCGCT CTGCTGAAGC CAGTTACCTT CGGAAAAAGA GTTGGTAGCT CTTGATCCGG 1560
CAAACAAACC ACCGCTGGTA GCGGTTTTTT TGTTTGCAAG CAGCAGATTA CGCGCAGAAA 1620
AAAAGGATCT CAAGAAGATC CTTTGATCTT TTCTACGGGG TCTGACGCTC AGTGGAACGA 1680
AAACTCACGT TAAGGGATTT TGGTCATGAG ATTATCAAAA AGGATCTTCA CCTAGATCCT 1740
TTTAAATTAA AAATGAAGTT TTAAATCAAT CTAAAGTATA TATGAGTAAA CTTGGTCTGA 1800
CAGTTACCAA TGCTTAATCA GTGAGGCACC TATCTCAGCG ATCTGTCTAT TTCGTTCATC 1860
CATAGTTGCC TGACTCCCCG TCGTGTAGAT AACTACGATA CGGGAGGGCT TACCATCTGG 1920
CCCCAGTGCT GCAATGATAC CGCGAGACCC ACGCTCACCG GCTCCAGATT TATCAGCAAT 1980
AAACCAGCCA GCCGGAAGGG CCGAGCGCAG AAGTGGTCCT GCAACTTTAT CCGCCTCCAT 2040
CCAGTCTATT AATTGTTGCC GGGAAGCTAG AGTAAGTAGT TCGCCAGTTA ATAGTTTGCG 2100
CAACGTTGTT GCCATTGCTA CAGGCATCGT GGTGTCACGC TCGTCGTTTG GTATGGCTTC 2160
ATTCAGCTCC GGTTCCCAAC GATCAAGGCG AGTTACATGA TCCCCCATGT TGTGCAAAAA 2220
AGCGGTTAGC TCCTTCGGTC CTCCGATCGT TGTCAGAAGT AAGTTGGCCG CAGTGTTATC 2280
ACTCATGGTT ATGGCAGCAC TGCATAATTC TCTTACTGTC ATGCCATCCG TAAGATGCTT 2340
TTCTGTGACT GGTGAGTACT CAACCAAGTC ATTCTGAGAA TAGTGTATGC GGCGACCGAG 2400
TTGCTCTTGC CCGGCGTCAA TACGGGATAA TACCGCGCCA CATAGCAGAA CTTTAAAAGT 2460
GCTCATCATT GGAAAACGTT CTTCGGGGCG AAAACTCTCA AGGATCTTAC CGCTGTTGAG 2520
ATCCAGTTCG ATGTAACCCA CTCGTGCACC CAACTGATCT TCAGCATCTT TTACTTTCAC 2580
CAGCGTTTCT GGGTGAGCAA AAACAGGAAG GCAAAATGCC GCAAAAAAGG GAATAAGGGC 2640
GACACGGAAA TGTTGAATAC TCATACTCTT CCTTTTTCAA TATTATTGAA GCATTTATCA 2700
GGGTTATTGT CTCATGAGCG GATACATATT TGAATGTATT TAGAAAAATA AACAAATAGG 2760
GGTTCCGCGC ACATTTCCCC GAAAAGTGCC ACCTGACCTC GACGGATCGG GAGATCTCCC 2820
GATCCCCTAT GGTGCACTCT CAGTACAATC TGCTCTGATG CCGCATAGTT AAGCCAGTAT 2880
CTGCTCCCTG CTTGTGTGTT GGAGGTCGCT GAGTAGTGCG CGAGCAAAAT TTCTGTGGAA 2940
TGTGTGTCAG TTAGGGTGTG GAAAGTCCCC AGGCTCCCCA GCAGGCAGAA GTATGCAAAG 3000
CATGCATCTC AATTAGTCAG CAACCAGGTG TGGAAAGTCC CCAGGCTCCC CAGCAGGCAG 3060
AAGTATGCAA AGCATGCATC TCAATTAGTC AGCAACCATA GTCCCGCCCC TAACTCCGCC 3120
CATCCCGCCC CTAACTCCGC CCAGTTCCGC CCATTCTCCG CCCCATGGCT GACTAATTTT 3180
TTTTATTTAT GCAGAGGCCG AGGCCGCCTC TGCCTCTGAG CTATTCCAGA AGTAGTGAGG 3240
AGGCTTTTTT GGAGGCCTAG GCTTTTGCAA AAAGCTAAGC TACAACAAGG CTCTGGCTAA 3300
CTAGAGAACC CACTGCTTAC TGGCTTATCG AAAGCTAGCT TAATACGACT CAATGAATCA 3360
GGGTGCAAAC AAGACGGTAT TAGACCGATA TTTACGGTTA GATATCCCGG ACCAGAAATG 3420
TCAAGCTATG TACATCTGGG TCGATGGAAC CGGCGAAAAC CTCCGCTCTA AGACCAGGAC 3480

ACTCAACTTT ACTCCTAAAT CTCCCAGTGA GCTGCCAATA TGGAATTTCG ATGGGTCATC 3540
AACGGGCCAG GCCGAACGGA GCAACAGTGA CGTGTACCTG TATCCAGTCG CTGTTTATCG 3600
AGATCCATTC AGGCTGGGTA ACAATAAGCT GGTCCTCTGT GAAACCTACA AATACAACAA 3660
GAAGCCTGCT GATACTAACC AGCGTTGGAA GTGTATGGAA GTAATGACAA GGGCAGCAGA 3720
CCAGCACCCA TGGTTCGGCA TGGAACAAGA ATATACTCTT TTGGACATTG ACAAACATCC 3780
CTTGGGTTGG CCCAAGAATG GCTATCCAGG CCCTCAGGGT CCCTATTACT GTGGTGTGGG 3840
TGCTAATAGG GTGTATGGGC GCGATGTGGT CGAGGCTCAC TACAGGGCGT GTCTTTGCGC 3900
TGGCATCAAC ATCTCTGGGG AGAACGCGAA AGTCATGCCG GCCCAATGGG AGTTCCAGGT 3960
TGGTCCGTGT GAAGGCATAA CCATGGGCGA CGACCTCTGG ATGGCTCGCT ACCTTCTTCA 4020
CAGGGTCGCT GAGGACTTTG ATGTTGTAGT AACACTCGAC CCCAAGCCAA TCCCTGGTGA 4080
CTGGAACGGC GCTGGAATGC ACACTAATTT CTCTACTGAA GCCATGCGTG GTCCCAATGG 4140
CATTCTGGAA ATTGAGAGTG CCATCGACAA ATTGTCGAAG GTTCATGAGA AACACATCAA 4200
GGCATACGAC CCACACGCAG GCAAGGATAA CGAAAGGCGC TTGACTGGTC ATTATGAAAC 4260
TTCCTCCATC CATGACTTTT CTGCAGGTGT GGCCAACCGT GGTGCCTCCA TCCGCATCCC 4320
CAGAGGAGTG GCTGAGGAGA AAACCGGCTA CCTGGAGGAC CGTCGCCCTT CCTCCAACGC 4380
TGACCCTTAT GTGGTGTCTG AGAGGCTTGT GCGTACCATC TGCCTGAACG AGCAGTGACT 4440
ATAGGGAGAC CCAAGCTGAC GCGCCCTGTA GCGGCGCATT AAGCGCGCCC GGGCTGGTGG 4500
TTACGCGCAG CGTGACCGCT ACACTTGCCA GCGCCCTAGC GCCCGCTCCT TTCGCTTTCT 4560
TCCCTTCCTT TCTCGCCACG TTCGCCGGCT TTCCCCGTCA AGCTCTAAAT CGGGGGCTCC 4620
CTTTAGGGTT CCGATTTAGT GCTTTACGGC ACCTCGACCC CAAAAAACTT GATTAGGGTG 4680
ATGGTTCACG TAGTGGGCCA TCGCCCTGAT AGACGGTTTT TCGCCCTTTG ACGTTGGAGT 4740
CCACGTTCTT TAATAGTGGA CTCTTGTTCC AAACTGGAAC AACACTCAAC CCTATCTCGG 4800
TCTATTCTTT TGATTTATAA GGGATTTTCT CTAGCTAGAG CTTGGCGTAA TCATGGTCAT 4860
AGCTGTTTCC TGTGTGAAAT TGTTATCCGC TCACAATTCC ACACAACATA CGAGCCGGAA 4920
GCATAAAGTG TAAAGCCTGG GGTGCCTAAT GAGTGAGCTA ACTCACATTA ATTGCGTTGC 4980
GCTCACTGCC CGCTTTCCAG TCGGGAAACC TGTCGTGCCA GCTGCATTAA TGAATCGGCC 5040
AACGCGCGGG GAGAGGCGGT TTGCGTATTG GGCGCTCTTC CGCTTCCTCG CTGTCCACCT 5100
CGCTGCGCTC GGTCGTTCGG CTGCGGCGAG CGGTATCAGC TCACTCAAAG GCGGTAATAC 5160
GGTTAGAGAT TTCGATTCCA CCGCCGCCTT CTATGAAAGG TTGGGCTTCG GAATCGTTTT 5220
CCGGGACGCC GGCTGGATGA TCCTCCAGCG CGGGGATCTC ATGCTGGAGT TCTTCGCCCA 5280
CCCCAACTTG TTTATTGCAG CTTATAATGG TTACAAATAA AGCAATAGCA TCACAAATTT 5340
CACAAATAAA GCATTTTTTT CACTGCATTC TAGTTGTGGT TTGTCCAAAC TCATCAATGT 5400
ATCTTATCAT GTCTGTATAC CGTCGACCTC AAGGCTTGAC CGACAATTGC ATGAAGACGC 5460
GTAATCTGCT TAGGGTTAGT TTTACAGGAT GGGGTCTCAT TTATTATTTA CAAATTCACA 5520
TATACAACAC CACCAGATCG CCTGGAGACG CCATCCACGC TGTTTTGACC TCCATAGAAG 5580
ACACCGGGAC CGATCCAGCC TCCGCGGCCG GGAACGGTGC ATTGGAACGC GGATTCCCCG 5640
TGCCAAGAGT GACGTAAGTA CCGCCTATAG AGTCTATAGG CCCACCCCCT TGGCTTCTTA 5700
TGCATGCTAT ACTGTTTTTG GCTTGGGGTC TATACACCCC CGCTTCCTCA TGTTATAGGT 5760

GATGGTATAG CTTAGCCTAT AGGTGTGGGT TATTGACCAT TATTGACCAC TCCCCTATTG 5820
GTGACGATAC TTTCCATTAC TAATCCATAA CATGGCTCTT TGCCACAACT CTCTTTATTG 5880
GCTATATGCC AATACACTGT CCTTCAGAGA CTGACACGGA CGCGTTTTGC GCTGCTTCGC 5940
GATGTACGGG CCAGATATAC GCGTTGACAT TGATTATTGA CTAGTTATTA ATAGTAATCA 6000
ATTACGGGGT CATTAGTTCA TAGCCCATAT ATGGAGTTCC GCGTTACATA ACTTACGGTA 6060
AATGGCCCGC CTGGCTGACC GCCCAACGAC CCCCGCCCAT TGACGTCAAT AATGACGTAT 6120
GTTCCCATAG TAACGCCAAT AGGGACTTTC CATTGACGTC AATGGGTGGA GTATTTACGG 6180
TAAACTGCCC ACTTGGCAGT ACATCAAGTG TATCATATGC CAAGTACGCC CCCTATTGAC 6240
GTCAATGACG GTAAATGGCC CGCCTGGCAT TATGCCCAGT ACATGACCTT ATGGGACTTT 6300
CCTACTTGGC AGTACATCTA CGTATTAGTC ATCGCTATTA CCATGGTGAT GCGGTTTTGG 6360
CAGTACATCA ATGGGCGTGG ATAGCGGTTT GACTCACGGG GATTTCCAAG TCTCCACCCC 6420
ATTGACGTCA ATGGGAGTTT GTTTTGGCAC CAAAATCAAC GGGACTTTCC AAAATGTCGT 6480
AACAACTCCG CCCCATTGAC GCAAATGGGC GGTAGGCGTG TACGGTGGGA GGTCTATATA 6540
AGCAGAGCTC GTTTAGTGAA CCGTCAGATC GCCTGGAGAC GCCATCCACG CTGTTTTGAC 6600
CTCCATAGAA GACACCGGGA CCGATCCAGC CTCCGCGGCC GGGAACGGTG CATTGGAACG 6660
CGGATTCCCC GTGCCAAGAG TGACGTAAGT ACCGCCTATA GAGTCTATAG GCCCACCCCC 6720
TTGGCTTCTT ATGCATGCTA TACTGTTTTT GGCTTGGGGT CTATACACCC CCGCTTCCTC 6780
ATGTTATAGG TGATGGTATA GCTTAGCCTA TAGGTGTGGG TTATTGACCA TTATTGACCA 6840
CTCCCCTATT GGTGACGATA CTTTCCATTA CTAATCCATA ACATGGCTCT TTGCCACAAC 6900
TCTCTTTATT GGCTATATGC CAATACACTG TCCTTCAGAG ACTGACACGG ACTCTGTATT 6960
TTTACAGGAT GGGGTCTCAT TTATTATTTA CAAATTCACA TATACAACAC CACCGTCCCC 7020
AGTGCCCGCA GTTTTTATTA AACATAACGT GGGATCTCCA CGCGAATCTC GGGTACGTGT 7080
TCCGGACATG GGCTCTTCTC CGGTAGCGGC GGAGCTTCTA CATCCGAGCC CTGCTCCCAT 7140
GCCTCCAGCG ACTCATGGTC GCTCGGCAGC TCCTTGCTCC TAACAGTGGA GGCCAGACTT 7200
AGGCACAGCA CGATGCCCAC CACCACCAGT GTGCCGCACA AGGCCGTGGC GGTAGGGTAT 7260
GTGTCTGAAA ATGAGCTCGG GGAGCGGGCT TGCACCGCTG ACGCATTTGG AAGACTTAAG 7320
GCAGCGGCAG AAGAAGATGC AGGCAGCTGA GTTGTTGTGT TCTGATAAGA GTCAGAGGTA 7380
ACTCCCGTTG CGGTGCTGTT AACGGTGGAG GGCAGTGTAG TCTGAGCAGT ACTCGTTGCT 7440
GCCGCGCGCG CCACCAGACA TAATAGCTGA CAGACTAACA GACTGTTCCT TTCCATGGGT 7500
CTTTTCTGCA GTCACCGTCC TTGACACGAA GCTTGCCACC ATGAATAAGC TGCTGTGCTG 7560
TGCCCTCGTG TTTCTCGATA TAAGCATTAA GTGGACTACC CAGGAGACAT TCCCTCCTAA 7620
GTATCTGCAC TATGACGAGG AGACAAGCCA TCAGCTGCTG TGCGATAAGT GTCCTCCTGG 7680
GACCTATCTC AAACAACATT GTACAGCCAA ATGGAAGACA GTCTGCGCTC CATGTCCTGA 7740
CCACTACTAC ACCGACTCTT GGCATACTAG CGACGAATGT CTGTATTGTT CACCCGTGTG 7800
CAAGGAGCTG CAATACGTGA AACAGGAATG CAATAGGACA CATAACCGCG TGTGTGAATG 7860
CAAAGAGGGC AGGTATCTGG AGATCGAATT TTGTCTGAAG CACCGGAGCT GCCCACCCGG 7920
CTTTGGAGTG GTCCAGGCCG GGACTCCCGA GAGAAACACT GTGTGCAAAA GATGCCCAGA 7980
CGGATTCTTT TCAAACGAGA CATCTTCTAA GGCACCATGT CGGAAGCACA CTAACTGTTC 8040

CGTCTTTGGG CTGCTGCTCA CCCAGAAGGG CAATGCCACC CACGATAATA TTTGCTCCGG 8100
AAACTCCGAA TCCACCCAAA AGTGCGGGAT AGATGTTACC CTCTGCGAAG AGGCATTCTT 8160
CCGCTTCGCT GTTCCTACCA AGTTCGACAA AACTCACACA TGCCCACCGT GCCCAGCTCC 8220
GGAACTCCTG GGCGGACCGT CAGTCTTCCT CTTCCCCCCA AAACCCAAGG ACACCCTCAT 8280
GATCTCCCGG ACCCCTGAGG TCACATGCGT GGTGGTGGAC GTGAGCCACG AAGACCCTGA 8340
GGTCAAGTTC AACTGGTACG TGGACGGCGT GGAGGTGCAT AATGCCAAGA CAAAGCCGCG 8400
GGAGGAGCAG TACAACAGCA CGTACCGTGT GGTCAGCGTC CTCACCGTCC TGCACCAGGA 8460
CTGGCTGAAT GGCAAGGAGT ACAAGTGCAA GGTCTCCAAC AAAGCCCTCC CAGCCCCCAT 8520
CGAGAAAACC ATCTCCAAAG CCAAAGGGCA GCCCCGAGAA CCACAGGTGT ACACCCTGCC 8580
CCCATCCCGG GATGAGCTGA CCAAGAACCA GGTCAGCCTG ACCTGCCTGG TCAAAGGCTT 8640
CTATCCCAGC GACATCGCCG TGGAGTGGGA GAGCAATGGG CAGCCGGAGA ACAACTACAA 8700
GACCACGCCT CCCGTGTTGG ACTCCGACGG CTCCTTCTTC CTCTACAGCA AGCTCACCGT 8760
GGACAAGAGC AGGTGGCAGC AGGGGAACGT CTTCTCATGC TCCGTGATGC ATGAGGCTCT 8820
GCACAACCAC TACACGCAGA AGAGCCTCTC CCTGTCTCCG GGTAAATAAT A 8871

In certain aspects, the present invention provides for a mammalian expression system for production of a polypeptide comprising the leading 215 amino acids of the human OPG followed by 227 amino acids of the Fc portion of the human Ig Gamma-1, optionally connected via a flexible linker. The expression system of the present invention comprises a mammalian cell harboring a recombinant mamalian expression plasmid for high expression of a polypeptide comprising the leading 215 amino acids of the human OPG followed by 227 amino acids of the Fc portion of the human Ig Gamma-1, optionally connected via a flexible linker.

In an example embodiment, the mammalian expression system of the present invention comprises Chinese hamster ovary cells (CHO-K1) harboring a plasmid comprising nucleotide sequence of SEQ ID NO. 3.

In certain aspects, the present disclosure provides for a method of treatment of a mammal effected by a disorder associated with bone resorption or remodeling.

### EXAMPLES

The following Examples illustrate the forgoing aspects and other aspects of the present invention. These non-limiting Examples are put forth so as to provide those of ordinary skill in the art with illustrative embodiments as to how the compounds, compositions, articles, devices, and/or methods claimed herein are made and evaluated. The Examples are intended to be purely exemplary of the invention and are not intended to limit the scope of what the inventor regard as his invention. Efforts have been made to ensure accuracy with respect to numbers (e.g., amounts, temperature, etc.) but some errors and deviations should be accounted for.

### Example 1: Construction of plasmids for expression of polypeptides of the present invention.

Initially, a first DNA fragment was chemically synthesized comprising DNA coding for hOPG amino acid residues 1 to 215, immediately followed by DNA coding for a leading fragment of hIgG1-Fc amino acids. At the 5'end of the first DNA fragment the hOPG coding sequence was preceded by Hind III restriction site, a Kozak sequence (GCCACC) together with its signal sequence. The hIgG1-Fc fragment coding DNA of the first DNA fragment at the 3' contained a Sac II restriction site.

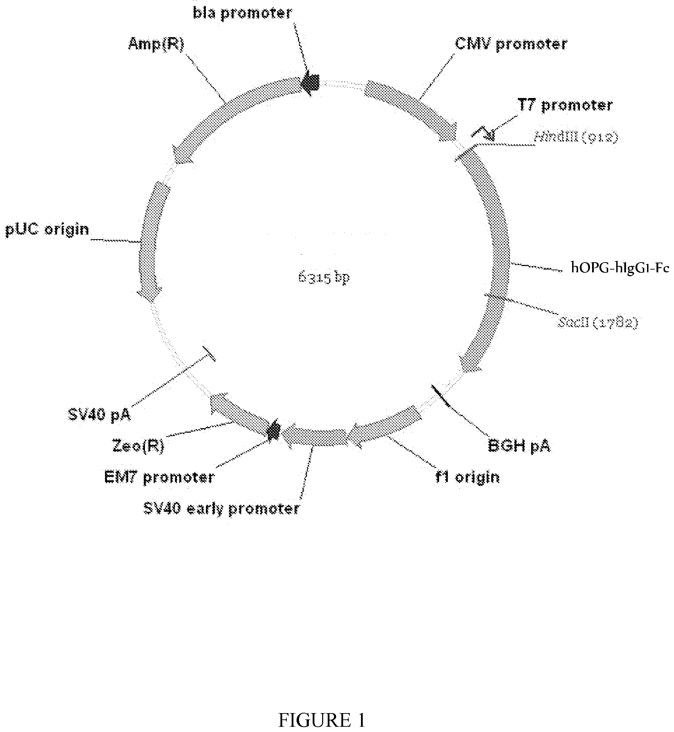

Subsequently, the synthetic gene was sub-cloned, utilizing the previously engineered Hind III and Sac II restriction sites, into a pCDNA4-Fc vector comprising DNA coding for the remaining hIgG1-Fc amino acids. The pCDNA4-Fc vector utilizes the cytomegalovirus (CMV) promoter to drive transcription of the gene of interest, and has been proven to generate high level expression in a wide variety of mammalian cell lines. The vector comprises the bGH polyadenylation and transcription termination sequences. The vector also contains a pUC origin of replication and a β-lactamase gene, which confers Ampicillin resistance, for supporting growth and selection in bacteria. **Figure 1** shows the pCDNA4-Fc plasmid map and annotated sequence.

Finally, the second DNA fragment comprising DNA coding for hOPG residues 1 to 215 and residues 103 to 329 of hIgG1-Fc was subcloned, utilizing the pCDNA4-Fc plasmid schematically shown in **Figure 1****,** into pKN002 mammalian expression vector for high expression of hOPG-hIgG1-Fc polypeptide of SEQ ID NO. 1. pKH002 vector utilizes the cytomegalovirus (CMV) promoter to drive transcription of the gene of interest, followed by the bGH polyadenylation and transcription termination sequence. The vector also contains a pUC origin of replication and β-lactamase gene, which confers ampicillin resistance, for supporting growth and selection in bacteria. Finally, pKN002 vector contains a gene for Glutamine synthetase - a selectable marker widely used to establish stable CHOK1 and NSO cell lines. **Figure 2** illustratively shows the map and annotated sequence of the resulting high expression pKH002-hOPG-hIgG1-Fc plasmid of SEQ ID NO. 3.

### Example 2: Generation of stable cell lines for expressing polypeptides of the present invention.

A stable clone of CHO-K1 cells overexpressing hOPG-hIgG1-Fc polypeptide of SEQ. ID 1 has been generated through standard protocols. The expression plasmid pKH002-hOPG-hIgG1-Fc described in Example 1 was used for generating stable cell lines for high expression of said polypeptide. Expression levels of said polypeptide in a plurality of clones were above 125 mg/L in a 7-day batch culture. One clonal cell line showed expression levels of about or over 125 mg/L in shake flask batch culture.

### Materials

Chinese hamster ovary cells (CHO-K1) were obtained as frozen stocks from ATCC (CCL-61™). The cells were adapted in house into a CD CHO media. Media and reagent were obtained from commercial source. Upon full adaptation, the cells were grown to high density for a few passages. The resulting cells were subcloned. One of the resulting clones with a doubling time under 20 hrs and good morphology was selected as parental cell line.

### Methods

A vial of CHO-K1 cells (7.5 x 10⁶ viable cells in a volume of 1.5 ml) was thawed into 20 ml of CD CHO medium supplemented with 4 mM L-glutamine to give a cell density of 3.75 x 10⁵ cells/ml in an Erlenmeyer flasks at 37°C/10% CO₂ on an orbital shaker platform at 80 to 100 rpm. Cells were subcultured every three to four days.

A research cell bank of 19 cryovials, each containing 7.5 x 10⁶ cells (passage 1) in 1.5 ml 50% fresh growth medium/50% conditioned medium containing 7.5% DMSO, were stored in liquid nitrogen. After further expansion, 50 cryovials each containing 7.5 x 10⁶ cells (passage 3) in 1.5 ml were frozen in liquid nitrogen (as described above) and have been used as a working cell bank.

Transfection of CHO-K1 cells with pKH002-hOPG-hIgG1-Fc plasmid described in Example 1was carried out by electroporation utilizing Gene Pulse Xcell instrument (BIO-RAD) using standard protocols. One-two days prior to electroporation, dilute CHO-K1 cells were transferred into fresh growth medium (CD CHO medium) so that they reached mid-log phase (generally about 2-3×10⁶cells/ml) on the day of the experiment. Following transfection, utilizing standard protocols, cell lines were adapted to suspension culture followed by measuring 50 ml culture growth curves. Cell count and viability were measured daily for each tested cell line. Production levels of the desired polypeptide were measure by two methods: ELISA; and test purifications by protein A column.

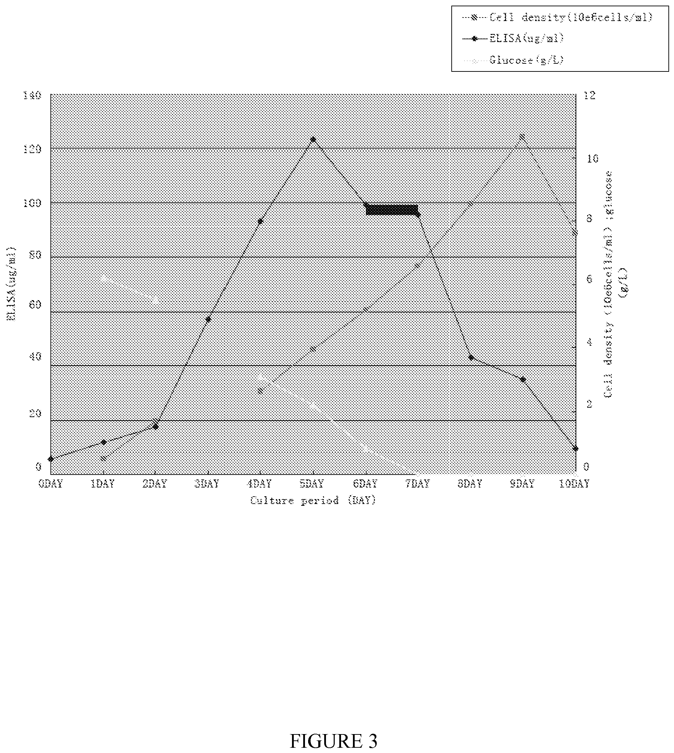

A number of transfections were carried out in CHO-K1 cells in the process of generation of potential hOPG-hIgG1-Fc expression cell line; a representative transfection growth curve is shown in **Figure 3** and the data is shown in Table 1. Supercoiled DNA was transfected into CHO-K1 cells under electroporation and selected with MSX. Cells were selected in 96 well plates as minipools and the productivity was assessed using SDS-PAGE and ELISA. Based on these analyses, two transfections were identified as producing the highest levels of hOPG-hIgG1-Fc, and these transfections will be characterized in more detail.

After initial selection in 96 well plates, 24 cell lines were scaled up to 24 well plates and then T25 flasks. The media were collected from the 24 well plates or T25 flasks, SDS-PAGE and ELISA were performed. Four lines that demonstrated good hOPG-hIgG1-Fc expression were transferred to suspension culture. The expression levels of two out of the above four cell lines were above 125mg/L in a 7 day batch culture, said two lines were selected a parental high expression cell lines.

The two parental high expression cell lines were plated for single cloning, seeding cells into 5 x 96 well plates. For both parental cell lines a plurality of clones were picked from 96 well plates and transferred to 24 well plates. Media were collected from confluent wells and assessed as before by SDS-PAGE and ELISA tests. Clones that did not produce desired results were discarded. A subset of the plurality of clones grew well in serum free suspension culture and 50 mL growth curves were assessed. All clones were additionally reanalyzed by SDS-PAGE and ELISA. From the subset of clones, the one with the highest productivity was chosen to be assessed in an additional set of 1L growth curves, confirming that the clone had the highest productivity and showed a significant improvement compared with its parental cell line.

The chosen highest production cell line was selected and thawed into CD-CHO. Growth curves on the cell line were assessed, and samples were collected daily for cell count, cell viability and hOPG-hIgG1-Fc productivity. Based on these studies, it was determined that selected highest productivity cell line was expressing hOPG-hIgG1-Fc polypeptide in amounts necessary to support commercial production. The yield of the polypeptide after purification was at least 125mg/L (without any production optimization).

**Table 1: A Representative Clonal Cell Line Growth curve**

| **Culture period (DAY)** | **Cell density (10⁶ cells/ml)** | **ELISA (ug/ml)** | **Glucose (g/L)** |
|---|---|---|---|
| 0 | 0.5 | | |
| 1 | 1 | 5.68 | 6.2 |
| 2 | 1.5 | 19.68 | 5.5 |
| 3 | 4.9 | | |
| 4 | 8 | 30.69 | 3.1 |
| 5 | 10.6 | 45.94 | 2.2 |
| 6 | 8.5 | 60.68 | 0.8 |
| 7 | 8.2 | 76.62 | 0 |
| 8 | 3.7 | 99.55 | 0 |
| 9 | 3 | 124.1 | 0 |
| 10 | 0.8 | 88.78 | 0 |

### Example 3: Purification of polypeptides of the present invention.

hOPG-hIgG1-Fc polypeptide of SEQ ID.1 was expressed in CHO-K1 essentially as described in foregoing Example 2. Cells were harvested and lysed utilizing well established protocols. After cell lysate clarification, the supernatant containing expressed hOPG-hIgG1-Fc polypeptide was first applied to a Protein A affinity column. The affinity purification step was carried out according to the procedure outlined in Table 2. The Protein A eluate containing hOPG-hIgG1-Fc was pH-adjusted to pH about 7.5 using 1 M Tris-HCl, pH 9.0. The conductivity was adjusted with deionized water (dH2O) as needed.

In order to reduce the contents of DNA, HCP, endotoxin and potential viral contaminants, the pH adjusted Protein A column eluate was further purified by anion-exchange chromatography (AIEX) utilizing Q Sepharose resin. The AIEX step is operated in a flowthrough mode according to the procedure outlined in Table 3.

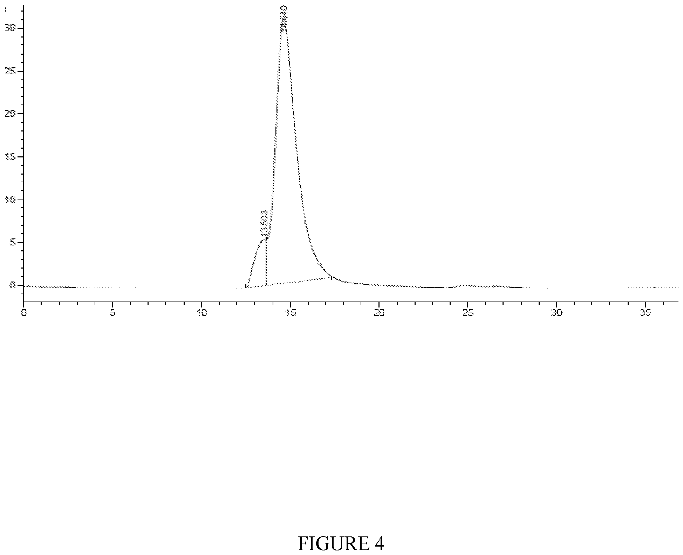

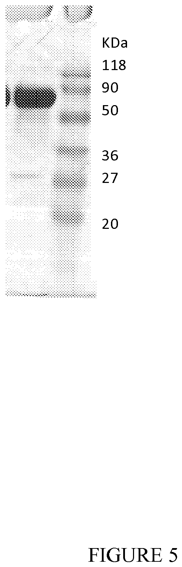

The AIEX flowthrough was analyzed by size-exclusion HPLC (SEC-HPLC) and SDS-PAGE (reducing). The results of the analysis are presented in **Figure 4** and **Figure 5****,** respectively.

The SEC-HPLC operational procedure is outlined in Table 4. The biologically relevant form of hOPG-hIgG1-Fc polypeptide is a homodimer linked via a C-terminal di-sulfide. Based on test runs with protein size markers, the molecular weight of the pre-shoulder peak (retention time RT=13.5 min) was about 378 KDa, indicating that it is likely representing a tetramer of hOPG-hIgG1-Fc.

**Table 2: The operational procedure of Protein A Affinity Chromatography**

| **Step** | **Buffer** | **Vol CV** | **Flow cm/h** |
|---|---|---|---|
| Rinse Before-use | dH2O | 3 | 150 |
| Equilibration | 10 mM PB, pH 6.0 | 3 | 150 |
| Sample Load | Cell harvest | - | 150 |
| Wash 1 | 10 mM PB, pH 6.0 | 3 | 150 |
| Wash 2 | 25 mM PB, 0.5 M NaCl, 5% Isopropanol pH 7.0 | 5 | 150 |
| Re-Equilibration | 10 mM PB, pH 6.0 | 3 | 150 |
| Elution | 20 mM Na-Citrate, pH 3.4 | 2.5 | 150 |
| Re-Equilibration | 10 mM PB, pH 6.0 | 3 | 150 |
| CIP | 0.1 M NaOH (contact time 15 min), reversed flow, CIP every 5 cycles | 3 | 100 |
| Rinse with NaCl | 1M NaCl | 3 | 150 |
| Rinse After-use | dH2O | 3 | 150 |
| Storage | 20% (v/v) Ethanol | 3 | 150 |

**Table 3: The operational procedure of AIEX**

| **Step** | **Buffer** | **Vol CV** | **Flow cm/h** |
|---|---|---|---|
| Rinse Before-use | dH2O | 3 | 150 |
| Recharge | 10 mM Tris-HCl, 1 M NaCl, pH 7.5 | 3 | 150 |
| Equilibration | 10 mM Tris-HCl, 50 mM NaCl, pH 7.5 | 3 | 150 |
| Sample Load | Prepared Q Load | - | 150 |
| Wash | 10 mM Tris-HCl, 50 mM NaCl, pH 7.5 | 3 | 150 |
| CIP | 1 M NaOH (contact time 1 hr), reversed-flow | 3 | 40 |
| Regeneration | 10 mM Tris-HCl, 1 M NaCl, pH 8.0 | 3 | 150 |
| Rinse After-use | dH2O | 3 | 150 |
| Storage | 20% (v/v) Ethanol | 3 | 150 |

**Table 4: The Operational Procedure of SEC-HPLC**

| | |
|---|---|
| Mobile Phase: | 20 mM phosphate, 300 mM NaCl, pH 7.4 |
| Flow Rate: | 0.5 mL/min |
| Column: | G2000 SWxl, 7.8mm×300mm, TOSOH Bioscience |
| Guard column: | TSK Guard SWx1,6.0mm×40mm, TOSOH Bioscience |
| Column Temperature: | 25 °C |
| Sampler temperature: | Rome temperature |
| Injection Volume: | 20-80 µg |
| Detector Wavelength: | 280 nm |
| Run Time: | 35 min |

To inquire whether the low pH elution conditions used in the Protein A affinity purification step could cause higher oligomer formation, a study was carried out to possibly correlate the eluate concentration and the oligomer content. An analytical Protein A column (2 mL) with MabSelect Sure resin was packed and used following the operational procedure outlined in Table 1. Four experiments were performed varying the column load volumes of the, i.e. 20, 50, 100 and 150 mL, respectively. The eluates collected were analyzed by SEC-HPLC to estimate the oligomer content. The results indicated that the oligomer was formed during the low-pH eluation, while the oligomer content had no correlation with the pool concentration.

Therefore, to reduce unwanted oligomerization, the following additional purification methods were investigated: hydrophobic interaction chromatography (HIC), cation exchange chromatography (CIEX), and hydroxylapatite (HA) chromatography.

For HIC, two resins, Phenyl- and Butyl- , and three mobile phase salts, NaCl, Na₂SO₄ and (NH₄)₂SO₄, were tested. Utilizing a Phenyl-based resin and NaCl based mobile phase demonstrated better result allowing to achieve a purity of > 97% and a yield of about 50%. Two CIEX resins were screened without showing superior results to those obtained from HIC.

Further tests utilizing hydroxylapatite chromatography (HAC) produced superior results. A HA column was packed with Macro-Prep Ceramic HA Type II 40µm (Bio-Rad) to a column volume (CV) of 10.4 mL (bed height 11 cm). Screening experiments were performed to identify best elution conditions (see Table 6). The starting material used was AIEX flowthrough after the initial two-step chromatography process. Initially, two batches of protein were prepared. The total protein expression levels for the first and the second batches were about 588 mg/L and 50 mg/mL, respectively. The purities and concentrations in AIEX flowthroughs from the two batches were comparable: about 92% and 2.5 mg/mL, respectively. The AIEX flowthrough was dialyzed against the HAC loading buffer before loading onto the HA column. All experiments were performed at a flow rate of 2 mL/min.

**Table 5: Screening Experiments on HAC**

| **Exp. No.** | **Loading Buffer (A)** | **Elution Buffer (B)** | **Elution Mode** |
|---|---|---|---|
| 1 | 5 mM PB, pH 6.5 | 500 mM PB, pH 6.5 | Linear gradient 0-100% B, 30 CV |
| 2 | 5 mM PB, pH 6.5 | 5 mM PB + 1 M NaCl, pH 6.5 | Linear gradient 0-100% B, 30 CV |
| 3 | 5 mM PB, pH 6.5 | 5 mM PB + 1 M NaCl, pH 6.5 | Stepwise 5%B; 10% B; 15% B; 20% B; 25% B; 30% B; 35% B; 100% B |
| 4 | 5 mM PB, pH 6.5 | 5 mM PB + 1 M NaCl, pH 6.5 | Stepwise, 30% B |
| 5 | 5 mM PB, pH 6.5 | 5 mM PB + 1 M NaCl, pH 6.5 | Stepwise, 25% B |
| 6 | 5 mM PB, pH 6.5 | 5 mM PB + 1 M NaCl, pH 6.5 | Stepwise. 20% B |
| 7 | 5 mM PB +10 mM MES, pH 6.5 | 5 mM PB +10 mM MES + 1 M NaCl, pH 6.5 | Stepwise, 20% B |

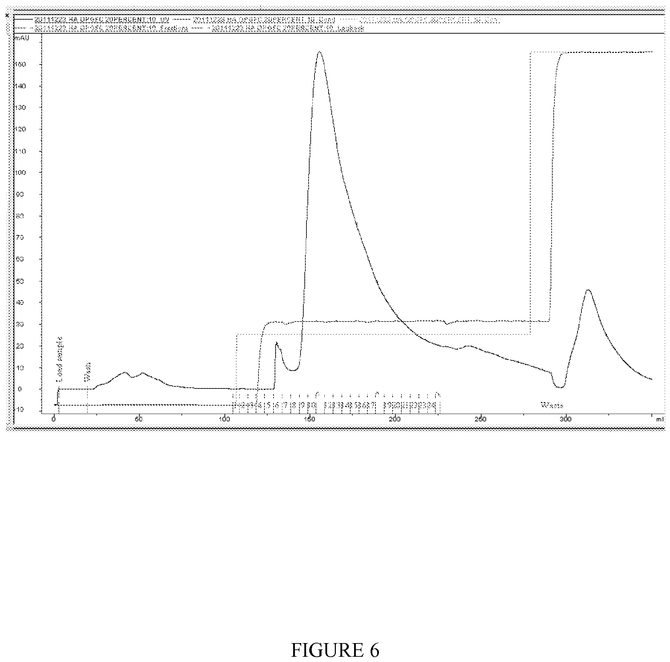

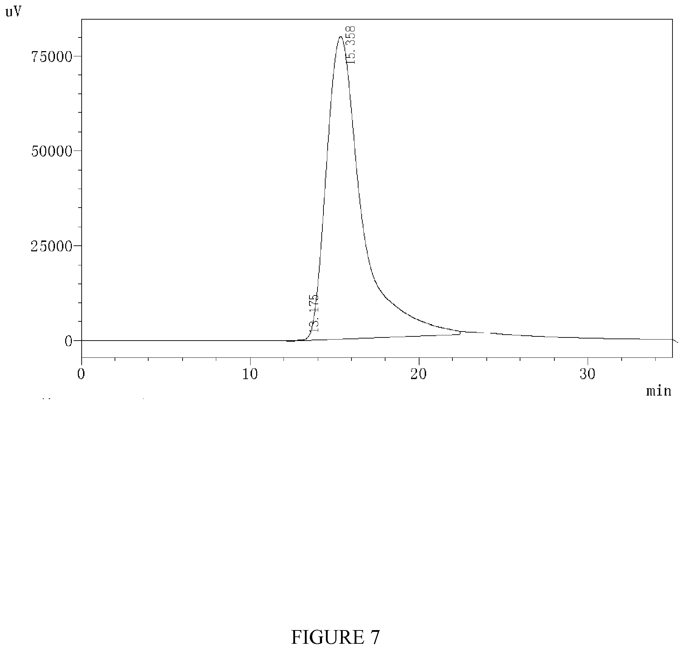

Among the experiments summarized in Table 5, experiment No. 6 produced superior results. The HAC chromatogram from experiment No. 6 is shown in **Figure 6****.** The pool of the main peak fractions from experiment No. 6 had purity over 99% (an example of SEC-HPLC analytical chromatogram of the HAC main peak fraction is shown in **Figure 7**) and estimated protein yield was 63%. Notably, experiment No. 5 also produced relatively good separation, however a lower protein yield of about 55%.

In an *in vitro* cell-based assay, polypeptide of SEQ ID NO. 1, which was expressed and purified essentially as described in this example, at a concentration of about 100 ng/ml completely blocked 50 ng/ml RANKL-induced osteoclast differentiation (RAW264.7 cells, EC50 about 50 ng/ml).

### Example 4: Toxicity study of hOPG-hIgG1-Fc polypeptide of SEQ ID NO. 1 for subcutaneous administration into mice.

Polypeptide of SEQ ID NO. 1 was expressed and purified essentially as described in the forgoing examples. For administration into animals, the polypeptide was formulated in the following buffer: 1% w/v Sucrose, 100 mM Sodium Chloride, 25 mM L-Arginine Hydrochloride, 25 mM Sodium Bicarbonate, pH 6.3. The dosing stock concentration used was 0.5 mg/mL of the polypeptide.

The SPF mice used in the study were randomly divided into 3 groups: formulation buffer control group, low dose group and high dose group, each group had 5 mice. The mice were anesthetized by intra-peritoneal injection of sodium pentobarbital, anesthetic dose was 30mg/kg. Back of mice was shaved to monitor the injection site. 20µL formulation buffer was administered to the control group by subcutaneous injection. 16mg/kg of the polypeptide was administered to the low dose group, and 32mg/kg of the polypeptide was administered to high dose group, responses at injection sites were observed and pictures taken at 15min, 6h, 24h, 72h and day 7.

In the study conducted, no acute reaction was observed in mice after subcutaneous administration of the formulation buffer alone, or of the polypeptide of SEQ ID. 1 in the doses of 16 and 32 mg/kg. No skin reaction or other abnormalities were observed for up to 7 days of observation.

### Example 5: Pharmacokinetics (PK) of hOPG-hIgG1-Fc polypeptide of SEQ ID NO. 1 after subcutaneous administration in mice.

Polypeptide of SEQ ID NO.1 was expressed and purified essentially as described in the forgoing examples. For administration into animals, the polypeptide was formulated in the following buffer: 1% w/v Sucrose, 100mM Sodium Chloride, 20 mM L-Arginine Hydrochloride, 25 mM Sodium Bicarbonate, pH 6.3. The dosing stock concentration used was 0.5 mg/mL of the polypeptide.

Fourteen female Balb/c nu/nu mice were randomized based on body weight into seven groups of two animals on Day 0 of the study. A single treatment of the polypeptide (5 mg/kg) was administered subcutaneously (dorsal) on Day 0 to all groups except mice in Group 1, which were bled via cardiac puncture for plasma preparation on Day 0 of the study. Blood samples were collected from mice via the orbital sinus in the remaining groups at various times through-out the study for preparation of plasma.

Body weights were recorded for all animals on the treatment day (Day 0) and then three times per week, including the termination day of each group.

Groups of mice were culled at specific time points for plasma preparation. Body weight changes were not measured in groups culled for sample collection at 0 hours and within 36 hours of dose administration. Most groups gained weight during the study period. Only Group 5 terminated at 7 days post-treatment failed to achieve an overall gain in body weight. No mice lost excessive body weight and no adverse clinical signs were reported during the study period.

Following the in-life phase of the study, plasma samples were analyzed by ELISA for Hu-Fc proteins. Quantification of Hu-Fc in mouse plasma samples by ELISA was used as a read-out for circulating levels of the polypeotide. The assay was performed on samples from all mice in the study.

The polypeptide was detected in the plasma of animals at 1 hour post-administration. One Phase Decay Model equation using Prism 5.0c (GraphPad Software Inc, La Jolla, CA, USA) was then used to determine pharmacokinetics of polypeptide of SEQ ID NO. 1 as detected by Hu-Fc ELISA. Peak circulating levels of Hu-Fc (Cmax) was determined to be 26.49 µg/mL, and time to peak circulating levels (Tmax) was 10 hours post treatment. The polypeptide levels decreased to near undetectable levels by three weeks post-administration, the final assessment point. The half-life (T1/2) was 83.52 hours and K0.0083 hr-1. Hu-Fc was not detected in the plasma of the untreated Group 1 animals. The results of the study are summarized in Table 6.

**Table 6: Mean Human-Fc Protein Concentration ± SEM (µg/mL) at each Time Post-Administration**

| Group | Treatment | Bleeding Schedule (time post-administration) | Mean Human-Fc Protein Concentration [µg/mL] | SEM |
|---|---|---|---|---|
| 1 | No treatment | 0 hours | 0.00 | -* |
| 2 | polypeptide of SEQ ID NO. 1 (5 mg/kg, Once only, s.c.) | 30 minutes^{^} | 0.00 | -* |
| 3 | | 1 hour^{^} | 1.96 | 1.04 |
| 4 | | 2 hours^{^} | 2.57 | 0.85 |
| 5 | | 4 hours^ | 15.89 | 1.45 |
| 6 | | 8 hours^ | 21.93 | 1.81 |
| 7 | | 10 hours^ | 26.49 | 0.97 |
| 2 | | 24 hours^{#} | 20.15 | 4.47 |
| 3 | | 36 hours^{#} | 16.10 | 0.84 |
| 4 | | 96 hours^{#} | 13.31 | 6.31 |
| 5 | | 7 days^{#} | 8.53 | 0.96 |
| 6 | | 14 days^{#} | 4.53 | -* |
| 7 | | 21 days^{#} | 1.09 | 0.25 |

| | | | | |
|---|---|---|---|---|
| SEM unable to be calculated as level of Hu-Fc was below detectable limit of ELISA for one of samples in Group. The Human-Fc Protein Concentration was determined by Prism Software based on the ^{^} Bleed via orbital sinus ^{#} Bleed via terminal cardiac puncture | | | | |

### Example 6: Determination of efficacy of hOPG-hIgG1-Fc polypeptide of SEQ ID NO. 1 against human breast cancer as a murine bone lytic model.

The murine bone lytic model used in the present example is well known in the art (e.g., see Arrington, S.A., et al., Bone. 2006 Mar;38(3):359-67). Polypeptide of SEQ ID NO.1 was expressed and purified essentially as described in the forgoing examples. For administration into animals, the polypeptide was formulated in the following buffer: 1% w/v Sucrose, 100mM Sodium Chloride, 20 mM L-Arginine Hydrochloride, 25 mM Sodium Bicarbonate, pH 6.3. The dosing stock concentration used was 0.5 mg/mL of the polypeptide.

Sixty female Athymic Nude-Foxn1^{nu} mice were each inoculated with 5 x 10⁵ MDA-MB-468 human breast cancer cells (in 5 µL) into the right tibia. Fourteen days post-inoculation, the mice were randomized by body weight into five groups of 12 (Day 0).

The mice in each group received subcutaneous treatment with either Vehicle (Formulation buffer) or polypeptide of SEQ ID NO.1 (1, 5 or 10 mg/kg), or intravenous treatment with Zometa (Zoledronic Acid) (0.1 mg/kg). Treatments were administered three times weekly, beginning on Day 0, and were continued for six weeks.

Body weight was recorded for all animals on the first treatment day (Day 0) and then three times per week, including the termination day of the study (48 hours post-final treatment, 42 days post-initial treatment). There was significant (p<0.05) mean body weight gain in all groups during the course of the study. No adverse clinical signs, including excess body weight loss, were observed in mice receiving treatment with polypeptide of SEQ ID NO.1 or Zometa.

Treatment with polypeptide of SEQ ID NO.1 at all doses and Zometa resulted in significant (p<0.05) inhibition of bone lysis assessed in X-ray images of tibiae taken at both six and eight weeks post-inoculation, compared with Vehicle. Although there was little or no evidence of bone lysis, the inoculated leg muscle appeared enlarged on the X-ray image taken at six weeks post-inoculation from one mouse receiving polypeptide of SEQ ID NO.1 at 10 mg/kg and two mice receiving Zometa. At eight weeks post-inoculation, the incidence of enlarged leg muscle increased in all groups. All mice with enlarged leg muscle receiving polypeptide of SEQ ID NO.1 or Zometa had mild or no evidence of bone lysis at eight weeks post-inoculation. X-ray images from all mice are presented,

Treatment with polypeptide of SEQ ID NO.1 at 1 mg/kg and Zometa resulted in significant (p<0.05) inhibition of bone lysis compared with Vehicle, indicated by bone density parameters assessed by microCT scan of inoculated tibiae excised at termination of the study. Representative two-dimensional and three-dimensional microCT scan images from one mouse in each of the groups analyzed for microCT are presented.

### Materials and Methods

All materials for the study were obtained from commercial sources. MDA-MB-468 human breast tumor cells were sourced from American Type Culture Collection (ATCC) (Rockville, MD, USA). MDA-MB-468 human breast tumor cells (Passage 5 from working stock) were cultured in RPMI 1640 cell culture medium, supplemented with 10% FBS, 1% Glutamax and 1% penicillin-streptomycin. The cells were harvested by trypsinization, washed twice in HBSS and counted. The cells were then resuspended in HBSS to a final concentration of 1x10⁸ cells/mL.

Prior to tumor inoculation, the injection site was liberally swabbed with alcohol. A 27G needle was introduced through the skin to drill a tunnel, 5-7mm in length (sufficient to accommodate 5 µL of cell suspension) from the plateau of the right hind tibia into the bone marrow channel. Using a 50 µL Hamilton-type syringe with a 27G needle, 5 µL of MDA-MB-468 cell suspension (5 x 105 cells) was discharged into the pre-formed tunnel. The treatment of mice began 14 days after MDA-MB-468 cell inoculation.

In total, 65 female mice (Mus musculus) were inoculated for the study, of which 60 were actually used in the study (in groups of 5, 12 groups in total). Animal body weight ranged at start of treatment from about 20.3 to about 27.8 g (mean 24.1 g) at inoculation. The animals were housed in groups of four in individually ventilated cages (Alternative Design Max, USA), three cages per group. The animals were kept in a controlled environment (targeted ranges: temperature 21 ± 3°C, humidity 30-70%, 15 air changes per hour), with a 12 hour light/dark cycle, and under barrier (quarantine) conditions. Temperature and relative humidity were monitored continuously. All animals were subjected to the same environmental conditions.

A standard certified commercial rodent diet (Teklad Global 18% Protein Rodent Diet, Harlan Laboratories, Inc, IN, USA) and tap water were provided to the animals ad libitum. Mortality checks were performed once daily in the morning during the study. Clinical signs (such as ill health and behavioral changes) were also recorded for all animals once daily. Body weights were recorded for all animals on the first treatment day (Day 0) and then three times per week, including the termination day of the study.

The following vehicle formulation was used in the study: 1% sucrose, 100 mM sodium chloride, 20 mM l-arginine hydrochloride, 25 mM sodium phosphate, pH6.3. The solution was stored at 4°C. The reference article Zometa (Zoledronic Acid) was used in the clinical formulation (0.8 mg/mL) and was stored at room temperature. The Test Article (polypeptide of SEQ ID NO.1) (45 mg/mL) was diluted in formulation buffer (1% sucrose, 100 mM sodium chloride, 20 mM 1-arginine hydrochloride, 25 mM sodium phosphate, pH6.3) to achieve the required concentration for dosing. Zometa® clinical formulation (0.8 mg/mL) was diluted in sterile saline to achieve the required concentration for dosing. The dosing solutions were prepared fresh on each day of dosing.

The dosing regimen used in this study is summarized in Table 7. Sixty mice were randomized by body weight on Day 0 of the study, 14 days post-inoculation, into five groups of 12 mice.

**Table 7: Animal Dosing regimen**

| Group | Compound | Treatment | Schedule |
|---|---|---|---|
| 1 | Vehicle (Formulation Buffer) | 10 mL/kg, s.c. | 3 times weekly for 6 weeks |
| 2 | polypeptide of SEQ ID NO.1 | 1 mg/kg in 10 mL/kg, s.c. | 3 times weekly for 6 weeks |
| 3 | polypeptide of SEQ ID NO.1 | 5 mg/kg in 10 mL/kg, s.c. | 3 times weekly for 6 weeks |
| 4 | polypeptide of SEQ ID NO.1 | 10 mg/kg in 10 mL/kg, s.c. | 3 times weekly for 6 weeks |
| 5 | Zometa | 0.1 mg/kg in 10 mL/kg, i.v. | 3 times weekly for 6 weeks |

Vehicle Control (Formulation Buffer; Group 1) and polypeptide of SEQ ID NO.1 (1, 5 and 10 mg/kg; Groups 2, 3 and 4, respectively) were administered by subcutaneous injection (s.c.) (Table 7). Zometa (0.1 mg/kg; Group 5) was administered by intravenous injection (i.v.) (Table 6). Treatments were administered three times weekly, beginning on Day 0 and continued for six weeks.

The Vehicle Control and Test Articles were administered in a dosing volume of 10 mL/kg. Each animal's body weight was measured immediately prior to dosing. The volume of dosing solution administered to each animal was calculated and adjusted based on individual body weight.

X-rays of both tibiae were performed on all mice at six and eight weeks post-inoculation (four and six weeks post-initial treatment). Visual assessment of bone lysis of the inoculated right tibiae was made from each X-ray image and a score was given to indicate severity of lysis and the presence of an enlarged leg muscle. The degree of lysis was awarded on a scale of 0 (no lysis) to 4 (very severe bone lysis). The presence of an enlarged leg muscle, as indicated by a bulging of the muscle tissue is awarded an additional score of 0.5.

The right inoculated tibia was excised from all mice in all groups at termination. Excised tibiae were preserved in 10% neutral buffered formalin for microCT analysis (Adelaide Microscopy, Adelaide, SA, Australia), comprising Total Bone Volume (TBV), Trabecular Bone Volume (Tb.BV), Trabecular Pattern Factor (Tb.Pf) and Structure Model Index (SMI).

Total Bone Volume (TBV) (mm3) is a measure of the total cortical and trabecular bone volume (Tb.BV) (mm3) within the volume of interest (VOI), where the VOI includes the cross-sectional region consisting of the cortical and trabecular bones. Trabecular Pattern Factor (Tb.Pf) (mm-1) is an inverse fragmentation index, which indicates the structural connectivity with specific application to the trabecular bone. A lower Tb.Pf value signifies better connected trabecular lattices while a higher Tb.Pf value indicates a more disconnected trabecular structure (i.e. more fragmentation / bone lysis). Structure Model Index (SMI) is an indicator of the relative prevalence of rods and plates in a 3D structure such as the trabecular bone. This parameter is important in osteolysis of the bone, which is characterized by a transition from plate-like (normal) to rod-like (degradation) structures. An ideal plate, cylinder and sphere have SMI values of 0, 3 and 4 respectively. The higher the value, the more damage there is. All statistical calculations were performed using Prism 5.0c (GraphPad Software Inc, La Jolla, CA, USA).

A paired t-test was used to determine if body weight changed significantly within a treatment group between Day 0 and termination of the study. Where the data did not pass the Normality Test, the Wilcoxon Matched Pairs Test was performed.

Comparison of bone lysis score assessed on X-ray images at six and eight weeks post-inoculation was made between all groups. As both data-sets failed the Normality Test, the Kruskal-Wallis One Way Analysis of Variance (ANOVA) on Ranks was performed. Multiple Comparison vs Control Procedure (Dunn's Method) was performed to compare each treatment with the Vehicle.

Comparison of bone lysis parameters assessed by microCT scan of excised inoculated tibiae was made between mice in Groups 1, 2 and 5. For normally distributed data, a One Way ANOVA was performed, and comparison between all groups made using Tukey's Multiple Comparison Test. Where the data failed the Normality Test, the Kruskal-Wallis One Way Analysis of Variance (ANOVA) on Ranks was performed. In this case, comparison between all groups was made using All Pairwise Multiple Comparison Procedure (Dunn's Method). A p value of less than 0.05 was considered significant.

### Results and Observations

No adverse clinical signs were observed in mice receiving treatment with polypeptide of SEQ ID NO.1 or Zometa (Groups 2-5). No mice receiving treatment with polypeptide of SEQ ID NO. 1 or Zometa (Groups 2-5) lost body weight in excess of 15% of initial weight during the study period. There was significant (p<0.05) mean body weight gain in all groups (Table 8).

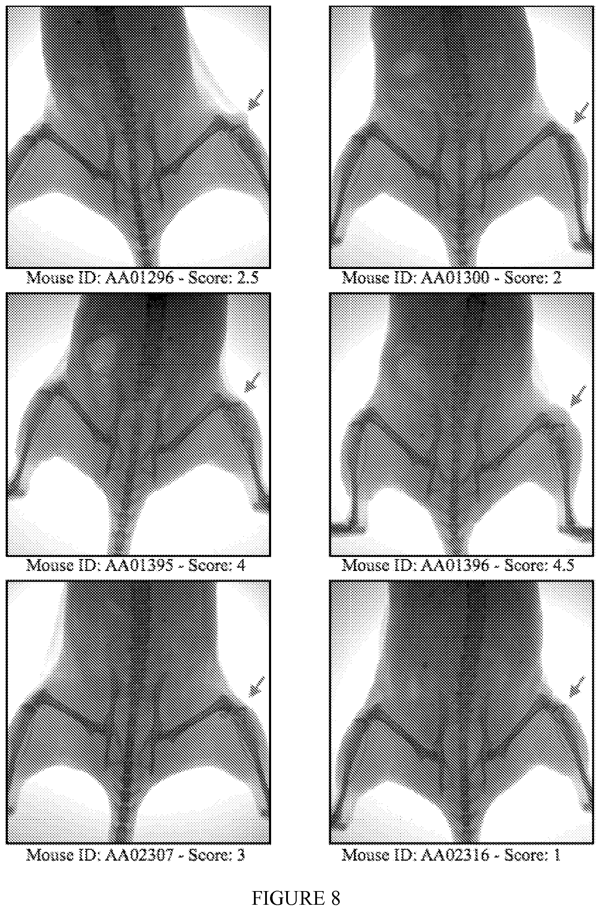

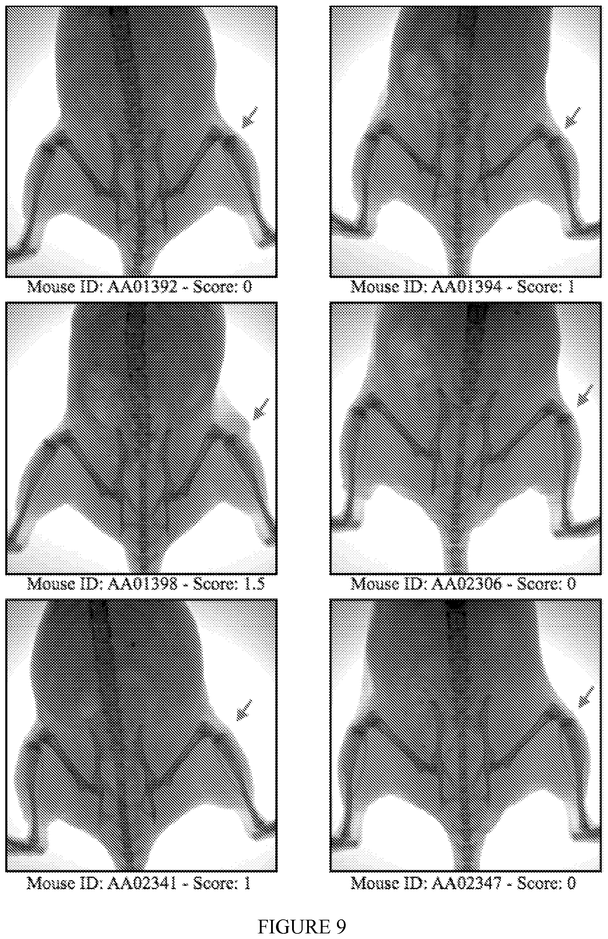

X-ray images of both tibiae were taken from all mice at Day 28 and 42 (six and eight weeks post-inoculation). Visual assessment of bone lysis of the inoculated right tibiae was made from each X-ray image and a score was given to indicate severity of lysis and the presence of an enlarged muscle on the inoculated leg (Table 9, and **Figure 8** and **Figure 9**). Treatment with polypeptide of SEQ ID NO.1 at all doses (1, 5 and 10 mg/kg; Groups 2, 3 and 4, respectively) and Zometa (Group 5) resulted in significant (p<0.05) inhibition of bone lysis assessed in X-ray images taken at both six and eight weeks post-inoculation compared with Vehicle control (Group 1).

**Table 8: Body Weight Changes and Survival Number for Each Group at the End of the Study**

| Group | Treatment | ***Host Response*** | | |
|---|---|---|---|---|
| | | Delta Body Weight ± SEM (g) | % Delta Body Weight | ***Survival (Number Alive*/*Total)*** |
| 1 | Vehicle (Formulation Buffer, s.c.) | 0.9 ± 0.7 | 3.8 | 11/12 |
| 2 | polypeptide of SEQ ID NO.1 (1 mg/kg, s.c.) | 1.8 ± 0.3 | 7.3 | 12/12 |
| 3 | polypeptide of SEQ ID NO.1 (5 mg/kg, s.c.) | 1.6 ± 0.3 | 6.6 | 12/12 |
| 4 | polypeptide of SEQ ID NO.1 (10 mg/kg, s.c.) | 2.3 ± 0.3 | 9.4 | 12/12 |
| 5 | Zometa (0.1 mg/kg, i.v.) | 1.2 ± 0.1 | 4.9 | 12/12 |

**Table 9: Mean X-ray Score of Bone Lysis in Inoculated Tibiae ± SEM at Six Weeks and Eight Weeks Post-Inoculation for Each Group**

| Group | Treatment | 6 weeks Post-Inoculation | | 8 weeks Post-Inoculation | |
|---|---|---|---|---|---|
| | | Mean X-Ray Score | SEM | Mean X-Ray Score | SEM |
| 1 | Vehicle (Formulation Buffer, s.c.) | 2.0 | 0.4 | 2.7 | 0.5 |
| 2 | polypeptide of SEQ ID NO.1 (1 mg/kg, s.c.) | 0.3 ^{a} | 0.1 | 0.3 ^{a} | 0.2 |
| 3 | polypeptide of SEQ ID NO.1 (5 mg/kg, s.c.) | 0.1 ^{a} | 0.1 | 0.3 ^{a} | 0.1 |
| 4 | polypeptide of SEQ ID NO.1 (10 mg/kg, s.c.) | 0.2 ^{a} | 0.1 | 0.5 ^{a} | 0.1 |
| 5 | Zometa (0.1 mg/kg, i.v.) | 0.2 ^{a} | 0.1 | 0.5 ^{a} | 0.2 |

| | | | | | |
|---|---|---|---|---|---|
| ^{a}: significantly different to Vehicle (Group 1) (p<0.05, Kruskal-Wallis One-Way ANOVA on Ranks) | | | | | |

### Score Legend:

- 0: No bone lysis
- 1: Onset/mild bone lysis
- 2: Moderate bone lysis
- 3: Severe bone lysis
- 4: Very severe bone lysis Enlarged muscle - score + 0.5

**Table 10: Mean Bone Lysis Parameters at Termination ± SEM for Each Parameter for Groups 1, 2 and 5, Measured by MicroCT Scan**

| Group | Treatment | | Total Bone Volume (TBV)(mm³) | Trabecular Bone Volume 3 (Tb.BV)(mm³) | Trabecular Pattern Factor (Tb.Pf) (mm⁻⁻¹) | Structure Model Index (SMI) |
|---|---|---|---|---|---|---|
| 1 | Vehicle (Formulation Buffer, s.c.) | Mean | 4.112 | 0.242 | 26.148 | 2.732 |
| | | SEM | 0.357 | 0.108 | 2.846 | 0.112 |
| 2 | polypeptide of SEQ ID NO.1 (1 mg/kg, s.c ) | Mean | 5.580 ^{a} | 0.494 ^{a} | 11.531 ^{a} | 1.888 ^{a} |
| | | SEM | 0.341 | 0.082 | 2.032 | 0.119 |
| 5 | Zometa (0.1 mg/kg, i.v.) | Mean | 6.799 ^{a} | 0.614 ^{a} | 10.458 ^{a} | 1.831 ^{a} |
| | | SEM | 0.370 | 0.093 | 1.547 | 0.115 |

| | | | | | | |
|---|---|---|---|---|---|---|
| ^{a}: significantly different to Vehicle (Group 1) (p<0.05, One Way ANOVA or Kruskal-Wallis One-Way ANOVA on Ranks) | | | | | | |

**Legend:**

| Parameter | Definition |
|---|---|
| Total Bone Volume (TBV) | Total cortical and trabecular bone volume (Tb.BV) within the volume of interest. |
| Trabecular Pattern Factor (Tb.Pf) | Fragmentation index; An inverse index of connectivity with specific application to the trabecular bone. A lower Tb.Pf signifies better connected trabecular lattices while higher Tb.Pf means a more disconnected trabecular structure (i.e. more lysis). |
| Structure Model Index (SMI) | An indicator of the relative prevalence of rods and plates in a 3D structure such as the trabecular bone. This parameter is important in osteolysis of the bone, which is characterized by a transition from plate-like (normal) to rod-like (degradation) structures. An ideal plate, cylinder and sphere have SMI values of 0, 3 and 4 respectively. The higher the value, the more damage there is. |

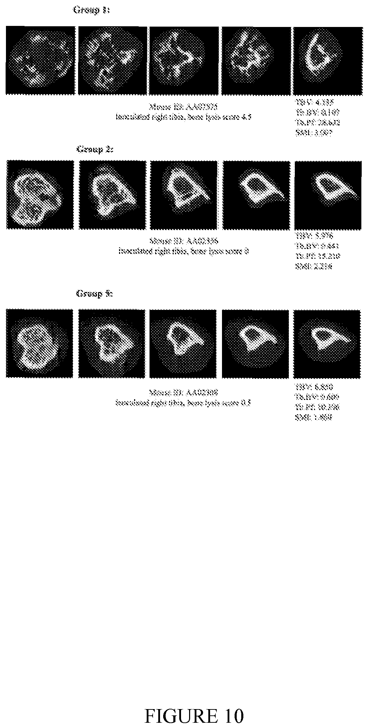

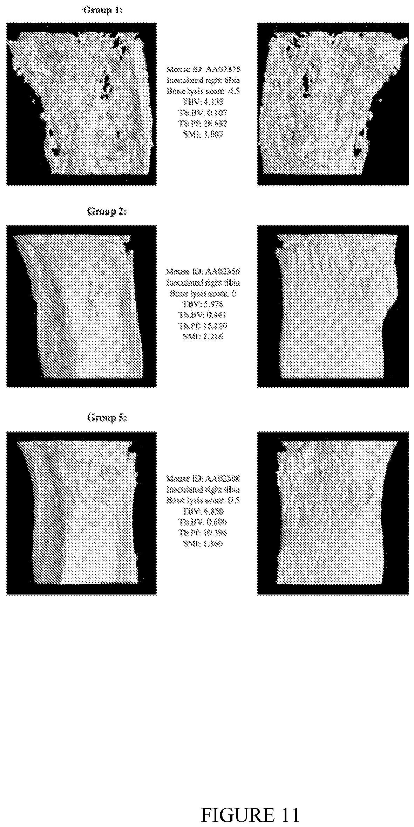

MicroCT scan parameters for assessment of bone lysis included a 3-dimensional morphometric analysis comprising Total Bone Volume (TBV), Trabecular Bone Volume (Tb.BV), Trabecular Pattern Factor (Tb.Pf) and Structure Model Index (SMI). The data is presented in Table 10. Representative images of microCT scans from one animal in each of the groups analyzed are presented in **Figure 10** and **Figure 11****.** Treatment with both polypeptide of SEQ ID NO.1 at 1 mg/kg (Group 2) and Zometa (Group 5) resulted in significant (p<0.05) inhibition of bone lysis compared with Vehicle control (Group 1), indicated by all microCT scan parameters (significantly higher TBV and Tb.BV, and significantly lower Tb.Pf and SMI).

It therefore follows that polypeptide of SEQ ID NO. 1 (1, 5 and 10 mg/kg, s.c. 3x per week for 6 weeks) was efficacious in preventing bone lysis induced by MDA-MB-468 human breast cancer cells, inoculated in the tibiae of female Athymic Nude-Foxn1^{nu} mice. This efficacy, measured by X-ray imaging at six and eight weeks post-inoculation and microCT scan of tibiae excised at termination (eight weeks post-inoculation) did not appear to be dose dependent. Likewise the reference compound for these studies, Zometa (0.1 mg/kg, i.v. 3x per week for 6 weeks) also demonstrated efficacy.

"Zometa" is a registered trademark of Novartis AG Corporation, Switzerland. "Prolia" and "Xgeva" are registered trademarks of Amgen Inc., a Delaware Corporation.

While specific embodiments of the subject matter have been discussed, the above specification is illustrative and not restrictive. Many variations will become apparent to those skilled in the art upon review of this specification and the claims below. The full scope of the invention should be determined by reference to the claims.

## Claims

1. A polypeptide that binds human RANKL, said polypeptide comprising:
a first amino acid sequence comprising amino acids 1 through 215 of human osteoprotegerin (GenBank: U94332.1); and
a second amino acid sequence comprising amino acids 103 through 329 of human immunoglobulin gamma-1 Fc (GenBank: J00228.1),
wherein said polypeptide is capable of inhibiting RANKL-induced osteoclast differentiation in an in vitro cell-based assay with an EC50 of 50 ng/ml or to no more than 10% at a concentration of 100 ng/ml.

2. The polypeptide of claim 1, said polypeptide comprising the amino acid sequence of SEQ ID NO. 1.

3. A therapeutic composition, the composition comprising a polypeptide according to claim 1 or 2 comprising a first amino acid sequence comprising amino acids 1 through 215 of human osteoprotegerin (GenBank: U94332.1) and a second amino acid sequence comprising amino acids 103 through 329 of human immunoglobulin gamma-1 Fc (GenBank: J00228.1), wherein said polypeptide is purified to a homo-dimeric form in solution to no less than 99%.

4. The therapeutic composition of claim 3, wherein:
a) the half-life of said polypeptide in systemic circulation in mice after a subcutaneous administration at a dose of 5 mg/kg is at least 80 hours; or
b) said composition is capable of inhibiting bone lysis induced by human breast cancer in a murine bone lytic model for at least 6 weeks in a course of subcutaneous administration of said composition at a dose of no more than 1 mg/kg three times weekly.

5. The therapeutic composition according to claim 3 or 4, wherein the polypeptide comprises the amino acid sequence of SEQ ID NO. 1.

6. An isolated nucleic acid encoding a polypeptide according to claim 1 or 2.

7. The nucleic acid of claim 6, wherein the encoded polypeptide comprises the amino acid sequence of SEQ ID NO. 1.

8. The nucleic acid of claim 6 or 7, wherein the codon usage is optimized for high expression of said polypeptide in a mammalian cell, optionally wherein the nucleic acid sequence comprises the sequence of SEQ ID NO. 2.

9. The nucleic acid of claim 6 to 8, wherein said nucleic acid is comprised in an expression vector.

10. A heterologous expression system, the expression system harboring an expression vector comprising a nucleic acid sequence encoding a polypeptide according to claim 1 or 2.

11. The expression system of claim 10, wherein the encoded polypeptide comprises the amino acid sequence of SEQ ID NO. 1.

12. The expression system of claim 10 or 11, wherein said expression vector is harbored in a mammalian cell such as a CHO cell, optionally wherein the level of said polypeptide expression is at least 125 mg per liter of cell culture.

13. A composition comprising a polypeptide according to claim 1 or 2 for use in the treatment or prevention of a disease associated with bone remodeling.

14. The composition for use according to claim 13, wherein said disease is selected from the group consisting of: a carcinoma; a breast cancer; a prostate cancer; multiple myeloma; a bone sarcoma; bone metastases due to solid tumors; osteoporosis; rheumatoid arthritis; and psoriatic arthritis.

## Patentansprüche

1. Ein Polypeptid, das humanes RANKL bindet, wobei das Polypeptid umfasst:
eine erste Aminosäuresequenz, umfassend die Aminosäuren 1 bis 215 von humanem Osteoprotegerin (GenBank: U94332.1); und
eine zweite Aminosäuresequenz, umfassend die Aminosäuren 103 bis 329 von humanem Immunglobulin gamma-1 Fc (GenBank: J00228.1),
wobei das Polypeptid in der Lage ist, RANKL-induzierte Osteoklasten-Differenzierung in einem zellbasierten in vitro-Assay zu inhibieren, mit einem EC50 von 50 ng/ml oder auf nicht mehr als 10% bei einer Konzentration von 100 ng/ml.

2. Das Polypeptid von Anspruch 1, wobei das Polypeptid die Aminosäuresequenz von SEQ ID NO. 1 umfasst.

3. Eine therapeutische Zusammensetzung, wobei die Zusammensetzung ein Polypeptid gemäß Anspruch 1 oder 2 umfasst, umfassend eine erste Aminosäuresequenz umfassend die Aminosäuren 1 bis 215 von humanem Osteoprotegerin (GenBank: U94332.1) und eine zweite Aminosäuresequenz umfassend die Aminosäuren 103 bis 329 von humanem Immunglobulin gamma-1 Fc (GenBank: J00228.1), wobei das Polypeptid in Lösung zu nicht weniger als 99% zu einer Homo-Dimer-Form aufgereinigt ist.

4. Die therapeutische Zusammensetzung von Anspruch 3, wobei:
a) die Halbwertszeit des Polypeptids im systemischen Kreislauf in Mäusen nach subkutaner Verabreichung einer Dosis von 5 mg/kg mindestens 80 Stunden beträgt; oder
b) die Zusammensetzung in der Lage ist, von humanem Brustkrebs induzierte Osteolyse in einem murinen Osteolyse-Model für mindestens 6 Wochen zu inhibieren, im Zuge von subkutaner Verabreichung der Zusammensetzung mit einer Dosis von nicht mehr als 1 mg/kg dreimal wöchentlich.

5. Die therapeutische Zusammensetzung gemäß Anspruch 3 oder 4, wobei das Polypeptid die Aminosäuresequenz von SEQ ID NO. 1 umfasst.

6. Eine isolierte Nukleinsäure, die ein Polypeptid gemäß Anspruch 1 oder 2 kodiert.

7. Die Nukleinsäure von Anspruch 6, wobei das kodierte Polypeptid die Aminosäuresequenz von SEQ ID NO. 1 umfasst.

8. Die Nukleinsäure von Anspruch 6 oder 7, wobei die Codon-Verwendung für hohe Expression des Polypeptids in einer Säuger-Zelle optimiert ist, wobei die Nukleinsäuresequenz optional die Sequenz von SEQ ID NO. 2 umfasst.

9. Die Nukleinsäure von Anspruch 6 bis 8, wobei die Nukleinsäure in einem Expressionsvektor enthalten ist.

10. Ein heterologes Expressionssystem, wobei das Expressionssystem einen Expressionsvektor beinhaltet, der eine Nukleinsäuresequenz umfasst, die ein Polypeptid gemäß Anspruch 1 oder 2 kodiert.

11. Das Expressionssystem von Anspruch 10, wobei das kodierte Polypeptid die Aminosäuresequenz von SEQ ID NO. 1 umfasst.

12. Das Expressionssystem von Anspruch 10 oder 11, wobei der Expressionsvektor in einer Säuger-Zelle, etwa einer CHO-Zelle, enthalten ist, wobei der Gehalt der Polypeptid-Expression optional mindestens 125 mg pro Liter Zellkultur ist.

13. Eine Zusammensetzung, umfassend ein Polypeptid gemäß Anspruch 1 oder 2 zur Verwendung bei der Behandlung oder Prävention einer Krankheit, die mit Knochen-Remodellierung assoziiert ist.

14. Die Zusammensetzung zur Verwendung gemäß Anspruch 13, wobei die Krankheit ausgewählt ist aus der Gruppe bestehend aus: einem Karzinom; einem Brustkrebs; einem Prostatakrebs; multiplem Myelom; einem Knochen-Sarkom; Knochenmetastasen aufgrund von soliden Tumoren; Osteoporose; rheumatoider Arthritis; und Psoriasis-Arthritis.

## Revendications

1. Polypeptide qui se lie à RANKL humain, ledit polypeptide comprenant :
une première séquence d'acides aminés comprenant les acides aminés 1 à 215 de l'ostéoprotégérine humaine (GenBank : U94332.1) ; et
une seconde séquence d'acides aminés comprenant les acides aminés 103 à 329 du Fc de l'immunoglobuline humaine gamma-1 (GenBank : J00228.1),
dans lequel ledit polypeptide est capable d'inhiber la différenciation des ostéoclastes induite par RANKL dans un essai cellulaire *in vitro* avec une CE50 de 50 ng/ml ou jusqu'à pas plus de 10 % à une concentration de 100 ng/ml.

2. Polypeptide selon la revendication 1, ledit polypeptide comprenant la séquence d'acides aminés de SEQ ID NO. 1.

3. Composition thérapeutique, la composition comprenant un polypeptide selon l'une des revendications 1 ou 2, comprenant une première séquence d'acides aminés comprenant les acides aminés 1 à 215 de l'ostéoprotégérine humaine (GenBank : U94332.1) et une seconde séquence d'acides aminés comprenant les acides aminés 103 à 329 du Fc de l'immunoglobuline humaine gamma-1 (GenBank : J00228.1), ledit polypeptide étant purifié jusqu'à une forme homo-dimère en solution à pas moins de 99 %.

4. Composition thérapeutique selon la revendication 3, dans laquelle :
a) la demi-vie dudit polypeptide dans la circulation systémique chez les souris après une administration sous-cutanée à une dose de 5 mg/kg est d'au moins 80 heures ; ou
b) ladite composition est capable d'inhiber la lyse osseuse induite par le cancer du sein humain dans un modèle lytique d'os murin pendant au moins 6 semaines dans le cadre d'une administration sous-cutanée de ladite composition à une dose de pas plus de 1 mg/kg trois fois par semaine.

5. Composition thérapeutique selon l'une des revendications 3 ou 4, dans laquelle le polypeptide comprend la séquence d'acides aminés de SEQ ID NO. 1.

6. Acide nucléique isolé codant pour un polypeptide selon l'une des revendications 1 ou 2.

7. Acide nucléique selon la revendication 6, dans lequel le polypeptide codé comprend la séquence d'acides aminés de SEQ ID NO. 1.

8. Acide nucléique selon l'une des revendications 6 ou 7, dans lequel l'usage de codons est optimisé pour une expression élevée dudit polypeptide dans une cellule de mammifère, facultativement dans lequel la séquence d'acide nucléique comprend la séquence de SEQ ID NO. 2.

9. Acide nucléique selon l'une des revendications 6 à 8, dans lequel ledit acide nucléique est compris dans un vecteur d'expression.

10. Système d'expression hétérologue, le système d'expression abritant un vecteur d'expression comprenant une séquence d'acide nucléique codant pour un polypeptide selon l'une des revendications 1 ou 2.

11. Système d'expression selon la revendication 10, dans lequel le polypeptide codé comprend la séquence d'acides aminés de SEQ ID NO. 1.

12. Système d'expression selon l'une des revendications 10 ou 11, dans lequel ledit vecteur d'expression est abrité dans une cellule de mammifère telle qu'une cellule CHO, facultativement dans lequel le taux de ladite expression de polypeptide est d'au moins 125 mg par litre de culture cellulaire.

13. Composition comprenant un polypeptide selon l'une des revendications 1 ou 2 pour une utilisation dans le traitement ou la prévention d'une maladie associée à un remodelage osseux.

14. Composition pour une utilisation selon la revendication 13, dans laquelle ladite maladie est choisie dans le groupe consistant en : un carcinome ; un cancer du sein ; un cancer de la prostate ; le myélome multiple ; un sarcome osseux ; des métastases osseuses dues à des tumeurs solides ; l'ostéoporose ; la polyarthrite rhumatoïde ; et l'arthrite psoriasique.
